# EUROPÄISCHE PATENTANMELDUNG

(11) **EP 3 263 115 A1**
(43) Veröffentlichungstag der Anmeldung: **03.01.2018**
(21) Anmeldenummer: 16177154.8
(22) Anmeldetag: 30.06.2016
(51) Int. Cl.: A61K 31/711, A61K 31/7115, A61K 31/712, A61K 45/06, A61P 31/04, C12N 15/11, C12N 15/113

(54) **ERFINDUNG BETREFFEND WIRKSTOFF GEGEN PSEUDOMONAS AERUGINOSA**

(71) Anmelder: Technische Hochschule Mittelhessen, 35390 Giessen (DE)
(72) Erfinder: Höfer, Frank, 63110 Rodgau (DE); Marquardt, Kay, 60327 Frankfurt (DE); Runkel, Frank, 35418 Buseck (DE)
(74) Vertreter: Stumpf, Peter

(57) **Zusammenfassung**

Die vorliegende Erfindung stellt einen neuartigen Therapieansatz zur Behandlung von Patienten mit *Pseudomonas aeruginosa* dar. Der neue Ansatz besteht in antisense-wirksamen katalytisch aktiven DNAzymen, die komplementär gegen konservierte Bereiche des Genoms von *Pseudomonas aeruginosa* gerichtet sind. Die erfindungsgenäßen DNAzyme sind sehr zielspezifisch auf die mRNA des *algD-*Gens gerichtet. Sie können Patienten in Form eines medizinischen Mittels sehr gut inhalativ verabreicht werden und spalten außergewöhnlich schnell am Zielort, sodass die Alginatsynthese, die für die Bildung des Biofilms verantwortlich ist, rasch unterbunden und blockiert wird. Somit steht Patienten mit cystischer Fibrose ein neues Mittel zur Verfügung, dass sehr schnell und zuverlässig wirksam ist.

## Beschreibung

### Beschreibung und Einleitung des allgemeinen Gebietes der Erfindung

Die Erfindung betrifft einen neuartigen Wirkstoff gegen das Bakterium *Pseudomonas aeruginosa*, welches vor allem bei Patienten der Cystischen Fibrose als Haupttodesursache bekannt ist.

Die Virulenz von *Pseudomonas aeruginosa* ist multifaktoriell und wird durch eine Reihe extrazellulärer Enzyme und anderer Substanzen bewirkt. Ihre Bedeutung für die Pathogenese menschlicher *Pseudomonas aeruginosa-*Infektionen ist vom jeweiligen Infektionstyp abhängig. Zwar werden therapeutisch seit langem Antibiotika zur Therapie gegen eine *Pseudomonas aeruginosa*-Infektion eingesetzt, jedoch weisen die Bakterien häufig mehrere Resistenzen auf, so dass Antibiotika nicht mehr zuverlässig wirksam sind. Die Dosis bisheriger Antibiotika wird derzeit aufgrund ihrer schwerwiegenden Nebenwirkungen limitiert. Bei Infektionen mit *Pseudomonas aeruginosa* kommt noch erschwerend hinzu, dass diese Bakterien zu einem bestimmten Zeitpunkt der Infektion einen zähen Biofilm in der Lunge bilden, der aus Alginat besteht. Alginat ist ein Polysaccharid, bestehend aus den beiden Uronsäuren α-L-Guluronsäure (GulUA) und β-D-Mannuronsäure (ManUA), welche1,4-glycosidisch in wechselndem Verhältnis zu linearen Ketten verbunden sind. Es bildet homopolymere Bereiche, in denen Mannuronsäure oder Guluronsäure als Blöcke vorliegen. Alginat neigt zur Gelbildung und damit zu einer erhöhten Viskosität. Das Alginat wird von *Pseudomonas aeruginosa* selber gebildet, wobei das Enzym AlgD (GDP-Mannose Dehydrogenase) bei der Synthese eine entscheidende Rolle spielt. Zahlreiche wissenschaftliche Studien zeigen, dass das körpereigene Immunsystem und auch Antibiotika diesen zähen Biofilm aus Alginat nicht durchdringen können.

Die Cystische Fibrose (CF) ist eine genetisch bedingte, schwere Stoffwechselerkrankung. Die Prävalenz liegt in Deutschland bei rund 8000 Menschen, wobei pro Jahr ungefähr 300 Kinder mit CF geboren werden. Die Ursache der Cystischen Fibrose (CF), auch Mukoviszidose genannt, ist eine Fehlfunktion von Chloridkanälen, wodurch die Zusammensetzung aller Sekrete von exokrinen Drüsen verändert wird. Diese Fehlfunktion beruht auf einer Mutation im CFTR Gen, das für das Cystische Fibrose Transmembran Regulator Protein kodiert. Vor allem in der Lunge, der Bauchspeicheldrüse, dem Dünndarm, den Gallenwegen, dem Reproduktionstrakt und den Schweißdrüsen werden zähflüssige Sekrete gebildet, die nur erschwert vom Körper abtransportiert werden können. Dadurch kommt es in den betroffenen Organen zu Funktionsstörungen unterschiedlichster Art. Für die Lebenserwartung und den Krankheitsverlauf ist aber vor allem der Zustand der Lunge ausschlaggebend. Der gebildete Schleim (Muccus) in den Bronchien von CF-Patienten ist deutlich zähflüssiger als im Vergleich zu gesunden Menschen. Dies führt zu chronischem Husten, Atemnot und häufig wiederkehrenden Lungenentzündungen, da sich auf dem Muccus Krankheitserreger wie *Pseudomonas aeruginosa, Staphylococcus aureus, Stentotrophomonas maltophila, Haermophilus influenzae*, *Exophiala dermatitis*, *Exophiala phaeomuriformis* u.a. ansiedeln, von denen *Pseudomonas aeruginosa* im zeitlichen Verlauf dominiert. Bei über 90% der CF-Patienten konnte eine Infektion mit *Pseudomonas aeruginosa* nachgewiesen werden. Eine Infektion mit *Pseudomonas aeruginosa* stellt die häufigste Todesursache bei CF-Patienten dar. Bei Patienten mit Cystischer Fibrose ist daher eine möglichst frühe, insbesondere vor der Bildung des Biofilms liegende, und vor allem wirksame und zuverlässige Behandlung gegen *Pseudomonas aeruginosa* entscheidend.

### Stand der Technik

Im Stand der Technik ist bekannt, bromhaltige Verbindungen als mikrobiozide Mittel zur Desinfektion von Oberflächen und zum Entkeimen von Wasser einzusetzen. Dazu offenbart z.B. DE 60213381 T2 ein bromhaltiges Mittel, das direkt auf den durch *Pseudomonas aeruginosa* hervorgerufenen Biofilm aufgetragen werden kann, den zähen Biofilm durchdringt und wirksam ist, indem es die Bakterien abtötet oder reduziert. Dieses Mittel ist aufgrund des Brom-Anteils nur zur äußeren Anwendung auf Oberflächen oder bei Gewässern geeignet und nicht als medizinisches Mittel gegen eine Infektion mit *Pseudomonas aeruginosa* einzuzsetzen. Es wirkt nicht spezifisch gegen *Pseudomonas aeruginosa*.

DE102010013834A1 offenbart genetisch modifizierte Phagen als therapeutisches Mittel gegen Pseudomonas Stämme PAK und PAO. Die filamentösen Phagen PF1 und PF3 sind mit antimikrobiell und antiinflammatorisch wirksamen Substanzen beladen und können die Pseudomonas-Stämme so infizieren, dass ein Platzen der Zellen und Freisetzung von Endotoxinen und anderen immunologisch aktiven Komponenten auf ein Minimum reduziert wird. Dieses Mittel kann auch prophylaktisch eingesetzt werden. Nachteilig ist, dass die gentechnisch veränderten Phagen nur gegen einige Pseudomonas-Stämme wirksam sind und die Verabreichung von gentechnisch veränderten Organismen an sich aus rechtlichen und ethischen Gründen schon problematisch ist.

Eine Lösung, die Bildung des bakteriellen Biofilms zu verhindert bzw. diesen zu zerstören offenbart DE102013020916A1. Hier wird als Mittel gegen eine *Pseudomonas aeruginosa* Infektion der intravenöse Einsatz einer normalen Kochsalzlösung angereichert mit Glycerin und Aminosäuren vorgeschlagen. Es wird beschrieben, dass Glycerin die Wirksamkeit von bekannten Antibiotika gegen den Biofilm unterstützt. Es ist also weiterhin die Therapie mit Antibiotika und der damit verbundenen Resistenzproblematik notwendig. Die Schrift stellt keinen Ersatz für die Antibiotika -Therapie dar.

DE102011108227A1 beschreibt als therapeutisches Mittel gegen *Pseudomonas aeruginosa*-Infektionen und auch als spezielle Behandlung bei cystischer Fibrose und chronisch obstruktiver Lungenerkrankung (englisch chronic obstructive pulmonary disease, Abkürzung: COPD) die Kombination von drei Wirkstoffen insbesondere Carbapenem, Polymyxin und Aminoglykosid, die inhalativ verabreicht werden können. Diese Therapie stellt eine optimierte Antibiotika-Therapie dar und ist nicht spezifisch gegen *Pseudomonas aeruginosa* gerichtet. Einige Bakterienstämme sind bereits bekannt als Carbapenem-resistent und können mit diesem Mittel nicht mehr bekämpft werden.

### Aufgabe

Aufgabe der vorliegenden Erfindung ist es die Nachteile im Stand der Technik zu beseitigen und ein Mittel zur Prophylaxe und Therapie von Infektionen mit *Pseudomonas aeruginosa* bereitzustellen, das wirksam ist und zielgerichtet die Alginatsynthese unterbindet.

### Lösung der Aufgabe

Diese Aufgabe wird erfindungsgemäß gelöst durch mindestens ein DNAzym gemäß SeqID 1-341 und dessen erfindungsgemäße Verwendung gemäß den Merkmalen des Anspruchs 1 und der abhängigen Ansprüche.

Bei der Erforschung der Pathogenese von *Pseudomonas aeruginosa,* insbesondere der Muccusproduktion und Alginatsynthese, wurde überraschenderweise eine Zielstruktur zur Wirkstoffentwicklung gefunden, welche nur in diesen Bakterien, nicht aber im Menschen vorkommt. Diese Zielstruktur ist die mRNA des *algD* Genes, die für das Protein AlgD codiert. Sie wurde als ideales Zielmolekül erkannt, da die Expression des *algD* Genes mit der Synthese des AlgD-Proteins einhergeht und unmittelbar mit der Alginatbildung und damit der Biofilmproduktion verbunden ist. Eine erste erfinderische Leistung war es, eine antisense Strategie zu finden, die auch in *Pseudomonas aeruginosa* funktioniert. Da in Prokaryoten wie *Pseudomonas aeruginosa* der RNA-induced-silencing-complex (RISC) nicht vorhanden ist, funktionieren herkömmliche antisense Strategien nur eingeschränkt. DNAzyme sind katalytisch aktive Einzelstrang DNA-Moleküle, die Ziel-Sequenzen von RNA-Molekülen spezifisch erkennen, komplementär binden und durch katalytische Aktivität spalten, wobei die Aktivität des gespaltenen RNA-Moleküls verloren geht oder herabgesetzt wird. DNAzyme bieten eine Möglichkeit auch ohne RISC die Translation eines Proteins zu blockieren, indem sie auf die mRNA des störenden Proteins wirken und dessen Expression entweder ganz oder teilweise hemmen.
Es wurde gefunden, dass die mRNA des *algD*-Gens von *Pseudomonas aeruginosa* ein sehr gutes Target für die Entwicklung eines DNAzyms gegen *Pseudomonas aeruginosa* darstellt. Eine Hemmung dieser mRNA unterbindet die Alginatsynthese und hemmt oder verlangsamt damit auch die Biofilmbildung. Die Hemmung der Alginatsynthese erzielt somit einen therapeutisch bedeutenden Effekt.

Es wurde gefunden, dass die Bakterien wenn die Alginatsynthese unterbunden ist, anfälliger gegenüber Antibiotika werden und somit leichter bekämpft werden können. Zudem können CF Patienten von einer besseren Atemfunktion profitieren, da der Gasaustausch durch die Alginate nicht mehr verstopft wird.

Alignment Analysen haben gezeigt, dass das AlgD-Protein und dessen Gentranskript für die Alginatsynthese ausschließlich in den Bakterien *Pseudomonas aeruginosa* und Azotobacter, sonst nur noch in Braunalgen vorkommen, es ist also sehr spezies-spezifisch. Das AlgD-Protein oder dafür kodierende mRNA kommt nicht im Menschen vor.
Es ist bekannt Antisense-Strategie zu nutzen, um krankheitsauslösende RNA-Moleküle im menschlichen oder tierischen Körper zu binden und zu blockieren.
Der Stand der Technik kennt als antisense wirksame Mittel siRNA oder "10-23"-DNAzyme, die hochspezifisch RNA-Zielstrukturen erkennen, binden und schneiden. Damit kann die Zielstruktur inaktiviert, gehemmt bzw. blockiert werden, so dass sie ihre krankheitsauslösende Funktion im Organismus nicht mehr erfüllt. Problematisch bei der Identifizierung von DNAzymen ist, dass zwar theoretisch eine Vielzahl von DNAzymen an die Zielstruktur binden müsste, tatsächlich aber nicht jedes DNAzym an der Zielstruktur gut bindet und zuverlässig schneidet. Um ein optimales DNAzym zu finden, müssen Spaltungsassays mit dem entsprechenden Zielmolekül durchgeführt werden.
Bisherige Spaltungsassays zur Bestimmung der enzymatischen Aktivität von DNAzymen waren ein zeitaufwändiger und kostenintensiver Prozess, bei dem unter anderem radioaktiver Abfall anfällt. Desweiteren unterliegen die Ergebnisse des Spaltungsassays hohen Mess-Schwankungen. Überraschenderweise ist es gelungen, ein einfaches, schnelles und kostengünstiges Verfahren zu entwickeln, in dem mittels Hochleistungsflüssigkeitschromatographie (englisch high performance liquid chromatography, *HPLC*) die Bestimmung der enzymatischen Aktivität von DNAzymen möglich wird. In diesem neuen Verfahren kommen keine radioaktiven Stoffe mehr zum Einsatz.

Erfindungsgemäß wird ein wirksames Mittel enthaltend mind. ein DNAzym ausgewählt aus der Gruppe Seq.ID 1-341 zur Verfügung gestellt, das spezifisch die mRNA des *algD*-Gens von *Pseudomonas aeruginosa* bindet und spaltet.
Dabei wurde eine spezielle Zielregion im Bakteriengenom gefunden und genutzt (Seq.ID 342), an der die Bindung des bzw. der DNAzyme zu einer Blockade oder Hemmung der Expression des AlgD-Proteins führt.
Ein Vorteil dieses spezifischen erfindungsgemäßen DNAzymes ausgewählt aus der Gruppe Seq.ID 1-341 ist, dass es nur in den bakteriellen Stoffwechsel von *Pseudomonas aeruginosa* eingreift, nicht aber in den menschlichen Stoffwechsel.

Die hohe Mutationsrate des Genoms von *Pseudomonas aeruginosa* kann zu Resistenzbildung führen auch wenn hoch-variable Stellen im Genom bei der Auswahl der Zielsequenz bewusst ausgespart wurden. Daher wird einer Resistenzbildung vorgebeugt, indem eine multi DNAzym Therapie empfohlen wird, wobei ein Mittel enthaltend mindestens zwei oder mehr der erfindungsgemäßen DNAzyme ausgewählt aus der Gruppe Seq.ID 1-341 eingesetzt wird. Weiterhin vorteilhafte Eigenschaften der spezifischen erfindungsgemäßen DNAzyme sind, dass sie die Alginat-Biosynthese in *Pseudomonas aeruginosa* wirksam blockieren, die ein zentraler Stoffwechselbestandteil ist. *Pseudomonas aeruginosa* kann das fehlende Protein und die damit verbundene enzymatische Funktion nicht durch andere Moleküle ersetzen. Als Folge der fehlenden oder gehemmten AlgD-Translation wird kein Alginat gebildet. Dadurch wird faktisch die Bildung und Aufrechterhaltung des bakteriellen Biofilms unterbunden. So hat letztlich eine Infektion mit *Pseudomonas aeruginosa* nicht mehr so drastische Auswirkungen auf den Menschen.

Da die erfindungsgemäßen DNAzyme sehr zielspezifisch und besonders wirksam sind, ist ihr therapeutischer Einsatz in einer sehr geringen Konzentration möglich. Da die DNAzyme an sich eine vergleichsweise sehr geringe Toxizität aufweisen, ist mit nur sehr wenigen bis gar keinen Nebenwirkungen bei der Applikation an einen Patienten z.B. der Lunge zu rechnen. Es wurde in eigenen Experimenten belegt, dass DNAzyme *in vivo* von körpereigenen DNasen allmählich zu Spaltprodukten in Form von Nukleotiden umgesetzt werden, die vom Körper wieder in den biologischen Stoff-Kreislauf eingebaut und wiederverwertet werden. *Pseudomonas aeruginosa* dagegen bildet keine DNasen, die DNAzyme abbauen könnten. Weiterhin sind die spezifischen erfindungsgemäßen DNAzyme ausgewählt aus der Gruppe Seq.ID 1-341 sogar zur Prophylaxe gegen eine Infektion mit *Pseudomonas aeruginosa* geeignet. Dies ist insbesondere für die Behandlung von CF-Patienten oder COPD Patienten von Bedeutung.

Die spezifischen erfindungsgemäßen DNAzyme ausgewählt aus der Gruppe Seq.ID 1-341 wirken in einem multiple-turnover d.h. ein einzelnes DNAzym vermag eine Mehrzahl von mRNAs des *algD*-Gens von *Pseudomonas aeruginosa* zu spalten. Dieser Effekt ist gegenüber anderen potentiellen Wirkstoffen ein besonderer Vorteil, die nur einen single-turnover aufweisen. Ein weiterer Vorteil der spezifischen erfindungsgemäßen DNAzyme ist, dass sie günstig nach Standardverfahren herstellbar sind und unter GMP-Bedingungen produziert werden. Dies ist insbesondere für die Durchführung klinischer Studien entscheidend.

DNAzyme weisen einen therapeutisch vielversprechenden Ansatz auf für die Behandlung von Krankheiten des menschlichen oder tierischen Körpers, um die verstärkte Expression von RNA Molekülen eines eingedrungenen Erregers wie z.B. *Pseudomonas aeruginosa* zu unterbinden. Voraussetzung ist jedoch, dass eine geeignete Zielstruktur bzw. die Zielsequenz für die Bindung gefunden wird, diese ausreichend identifiziert und zugänglich ist und die DNAzyme sowohl eine gute Bindeeigenschaft als auch eine katalytische Aktivität aufweisen.
10-23-DNAzyme weisen klassischerweise eine katalytische Domäne von 15 Nukleotiden auf, welche von zwei Substratbindungsdomänen (I und II) flankiert wird. Die Bindung an das RNA-Substrat erfolgt durch Basenpaarung gemäß den Watson-Crick Regeln über die Substratbindungsdomänen I und II.
Die spezifischen erfindungsgemäßen DNAzyme gegen die mRNA des *algD*-Gens sind vom gängigen 10-23 Molekül abgeleitet, wobei die beiden Bindungsarme nukleotidspezifisch für *algD* mRNA sind, nur diese mRNA binden und durch die katalytische Aktivität zerschneiden bzw. inaktivieren.

### Zielsequenz zur Bindung der erfindungsgemäßen DNAzyme

In eigener wissenschaftlicher Arbeit wurden 122 Isolate von *Pseudomonas aeruginosa* aus Patienten mit gesicherter Diagnose einer Cystischen Fibrose miteinander verglichen und insbesondere die mRNA des *algD*-Gens von *Pseudomonas aeruginosa* auf sehr konservierte Bereiche und gut zugängliche Spaltungsstellen für DNAzyme hin untersucht.
Überraschenderweise wurden dabei mehrere Abschnitte in der mRNA des AlgD-Gens von *Pseudomonas aeruginosa* identifiziert, die als Target geeignet sind. Die gesamte Zielsequenz umfasst einen Bereich von 1311 Nukleotiden (SeqID 342).

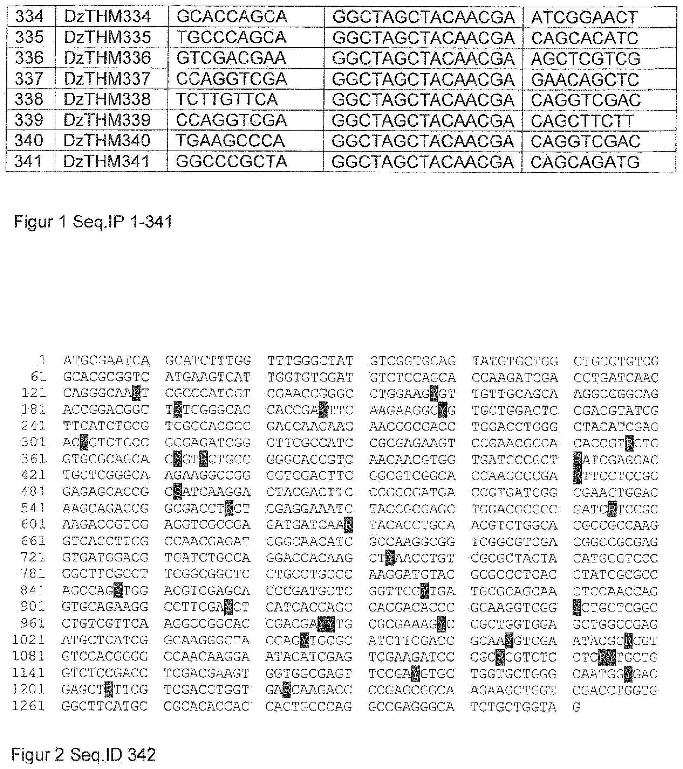

Figur 2 (SeqID 342) zeigt die ausgewählten Bereiche, die sich zwischen den variablen Regionen (schwarz hinterlegt) befinden, wobei die variablen Regionen weit genug auseinanderliegen. Die ausgewählten Bereiche weisen eine Purin-Pyrimidin Schnittstelle für das DNAzym auf.
Als besonders gute Bindestelle für DNAzym DzTHM95 wurde die Bindestelle Nukleotide 70-88 in der SeqID 342 ausgewählt.
Als besonders gute Bindestelle für DNAzym DzTHM290 wurde die Bindestelle Nukleotide 231-249 in der SeqID 342 ausgewählt.
Die erfindungsgemäßen DNAzyme sind spezifisch gegen diese Zielsequenzen gerichtet. Sie stellen somit ein wirksames Mittel zur Prophylaxe und Therapie von Infektionen mit *Pseudomonas aeruginosa* dar. Sie sind als medizinisches Mittel bei CF-Patienten oder COPD-Patienten geeignet.

Insbesondere weisen die erfindungsgemäßen DNAzyme ausgewählt aus der Gruppe Seq.ID 1-341 unerwartet positive Eigenschaften auf hinsichtlich
- Spezifische Bindung an die jeweilige Zielsequenz
- Schnelle und zuverlässige enzymatische Spaltungsaktivität an der Zielsequenz
- Starke Inaktivierung der mRNA des *algD*-Gens von *Pseudomonas aeruginosa*
- Verhinderung der Bildung der Alginatbiosynthese
- Verhinderung der Bildung des zähen Biofilms
Überraschenderweise ist es gelungen, ein einfaches, schnelles und kostengünstiges Verfahren zur Bestimmung der Funktionskinetik der DNAzyme zu entwickeln, in dem keine radioaktiven Stoffe mehr zum Einsatz kommen.

Mit diesem neuen Verfahren zur Bestimmung der enzymatischen Aktivität von DNAzymen mittels Hochleistungsflüssigkeitschromatographie (englisch high performance liquid chromatography, *HPLC*) wird die Kinetik der Spaltung der RNA durch die erfindungsgemäßen DNAzyme im Vergleich zu einem bekannten DNAzym und dessen Target (Dz13 und hgd40) detektiert und gezeigt.

| **Name** | ***v* in nM/min** |
|---|---|
| Vergleichs-DNAzym Dz13 | 17.51 ± 0.85 |
| Vergleichs-DNAzym hgd40 | 7.52 +- 0,90 |
| DzTHM95 | 29.63 ± 0.75 |
| DzTHM290 | 39.79 ± 1.12 |

### Aufbau der erfindungsgemäßen DNAzyme

Die spezifischen erfindungsgemäßen DNAzyme gegen die mRNA des *algD*-Gens sind ausgewählt aus der Gruppe Seq.ID 1-341. Sie umfassen jeweils eine zentrale katalytische Domäne von 15 Desoxyribonukleinsäuren mit der Sequenz **GGCTAGCTACAACGA**, welche von zwei Substratbindungsdomänen I und II flankiert wird. Die beiden Substratbindungsdomänen I und II sind wichtig für die katalytische Aktivität des DNAzyms bei der Spaltung der Zielsequenz durch De-Esterifikation. Sie erkennen die spezifische Zielsequenz an der mRNA des *algD-*Gens und binden über Watson-Crick Basenpaarung sehr nukleotidspezifisch.

Die erfindungsgemäßen DNAzyme erkennen die RNA Bereiche, binden diese Bereiche komplementär und schneiden diese. Damit wird die mRNA des *algD*-Gens inaktiviert, gehemmt oder blockiert, so dass sie ihre Replikation und damit die Bildung von AlgD Protein nicht mehr durchführen kann. So wird die gesamte Alginatsynthese unterbunden und der zähe Biofilm nicht mehr gebildet.

Die erfindungsgemäßen DNAzyme stellen ein wirksames Mittel für den medizinischen Einsatz zur Behandlung oder Prophylaxe einer Infektion mit *Pseudomonas aeruginosa* dar. Der medizinische Einsatz zur Prophylaxe und Behandlung erfolgt am Menschen und/oder Säugetier.

Insbesondere weisen die erfindungsgemäßen DNAzyme (DzTHM95 und DzTHM290) ausgewählt aus der Gruppe Seq.ID 1-341 unerwartet positive Eigenschaften u.a. eine sehr schnelle Spaltung auf.

| Seq.ID | DNAzym | Substratbindedomäne I | Katalytische Domäne | Substratbindedomäne II |
|---|---|---|---|---|
| 95 | DzTHM95 | CCACACCAA | GGCTAGCTACAACGA | GACTTCATG |
| 290 | DzTHM290 | GCAGATGAA | GGCTAGCTACAACGA | GATACGTCG |

In einer Spaltungskinetik, die mit HPLC gemessen wurde, zeigt sich, dass die erfindungsgemäßen DNAzyme die RNA-Zielsequenz sehr schnell und zuverlässig spalten, sie sind weiterhin optimal in ihrer Aktivität auf die Temperaturverhältnisse in der Lunge abgestimmt. Die Temperatur in der Lunge liegt bei ≤ 37°C.

Die Länge der Substratbindungsdomänen I und II umfasst jeweils mind. 8 Nukleotide. Von den theoretisch aus der Zielsequenz ableitbaren DNAzyme wurden diejenigen DNAzyme ausgewählt. Diese DNAzyme werden im Folgenden als spezifische erfindungsgemäße DNAzyme bezeichnet. Sie weisen eine überraschend gute Spaltungseigenschaft auf.
In einer Ausführung sind die Substratbindungsdomänen I und II vollständig komplementär zu der speziellen Zielsequenz an der mRNA des *algD*-Gens von *Pseudomonas aeruginosa.* Um dort zu binden und zu spalten, müssen die Substratbindungsdomänen I und II der erfindungsgemäßen DNAzyme jedoch nicht unbedingt vollständig komplementär sein. *In vitro* Untersuchungen zeigen, dass die erfindungsgemäßen DNAzyme mit einer Substratbindungsdomäne I oder II, die eine Homologie von ca. 90 % aufweisen, ebenfalls an die Zielsequenz binden und diese effizient spalten können.
Die erfindungsgemäßen DNAzyme können alternativ bis zu zwei Missmatching Nukleotide pro DNAzym aufweisen.

### Modifikationen

Das mindestens eine erfindungsgemäße DNAzym ausgewählt aus der Gruppe Seq.ID 1-341 wird alternativ durch mind. eine Modifikation gegenüber nukleolytischen Angriffen stabilisiert. Dies ist insbesondere für die Anwendung als medizinisches Mittel wichtig. Die Modifikation bewirkt, dass das stabilisierte DNAzym eine verbesserte katalytische Aktivität gegenüber dem jeweiligen RNA-Substrat aufweist. Als Modifikation wird eingesetzt:
Geschwindigkeitsbeschleunigende Modifikationen mit einem kovalent eingeführten Interkalator X, der eine Modifikation/Substitution des 3' Adenins in der katalytischen Domäne darstellt mit, wobei X sein kann:
   a) t-azo(trans)
   b) t-Pyr
   c) t-Anth
   d) t-Stl

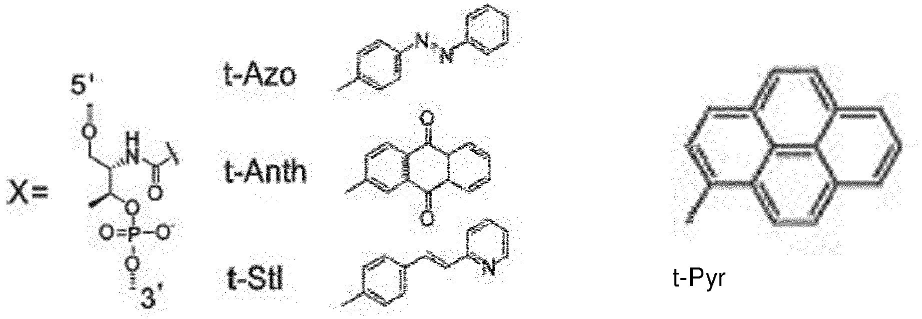

5' -CCACACCAA GACTTCATG- 3'
G X
G A
C G
T C
A A
G A
C T A C

modifiziertes DNAzym

Eine weitere Modifikation umfasst eine chemische Modifizierung eines Nukleotids. Darunter versteht der Fachmann, dass das modifizierte Nukleotid durch Entfernen, Anfügen oder Ersetzen einzelner oder mehrerer Atome oder Atomgruppen im Vergleich zu natürlich vorkommenden Nukleotiden verändert ist. Die chemische Modifikation umfasst beispielsweise die Ribose (z.B. 2'-O-Methyl-Ribose, sog. "Locked Nucleic Acid" (LNA)-Ribonukleotide und invertiertes Thymidin), die Phosphor(di)esterbindung (z.B. Phosphorthioate, Phosphoramidate, Methylphosphonate und Peptidnukleotide) und/oder die Base (z.B. 7-Deazaguanosin, 5-Methylcytosin und Inosin).

In einer besonders bevorzugten Ausführungsform sind die erfindungsgemäßen DNAzyme an mindestens einem Nukleotid der Substratbindungsdomäne I und/oder II modifiziert, insbesondere durch Phosphorthioat, invertiertes Thymidin, 2'-O-Methyl-Ribose oder LNA Ribonukleotide. Bei weiterhin bevorzugten DNAzymen ist ein Nukleotid oder sind mehrere Nukleotide der katalytischen Domäne modifiziert, insbesondere durch Phosphorthioat, invertiertes Thymidin, 2'-O-Methyl-Ribose oder LNA Ribonukleotide.
Eine bevorzugte Ausführung ist die Einführung einer 3'-3'-Inversion an einem Ende der erfindungsgemäßen DNAzyme. Der Begriff 3'-3'-Inversion bezeichnet eine kovalente Phosphatbindung zwischen den 3'-Kohlenstoffen des terminalen Nukleotids und des angrenzenden Nukleotids. Dieser Typ von Bindung steht im Gegensatz zu der normalen Phosphatbindung zwischen den 3' und 5' Kohlenstoffen von aufeinander folgenden Nukleotiden. Dementsprechend wird bevorzugt, dass das Nukleotid am 3'-Ende der an das 3'-Ende der katalytischen Domäne angrenzenden Substratbindungsdomäne invers ist. Zusätzlich zu den Inversionen können die DNAzyme modifizierte Nukleotide oder Nukleotid-Verbindungen enthalten. Modifizierte Nukleotide beinhalten z.B. N3'-P5'-Phosphoramidat Verbindungen, 2'-O-Methyl-Substitutionen und Peptid-Nukleinsäure-Verbindungen. Die Herstellung sämtlicher Modifikationen ist dem Fachmann auf diesem Gebiet geläufig und er findet Anleitungen zum Vorgehen im Stand der Technik.

In einer Ausführungsform ist die Modifikation in der katalytischen Domäne lokalisiert. In einer weiteren Ausführungsform ist die Modifikation in der Substratbindungsdomäne I und/oder II lokalisiert. In einer weiteren Ausführungsform ist die Modifikation in der katalytischen Domäne und in der Substratbindungsdomäne I und/oder II lokalisiert.

### Verwendung als Arzneimittel

Mindestens eines der erfindungsgemäßen DNAzyme ausgewählt aus der Gruppe Seq.ID 1-341 mit oder ohne Modifikation ist Bestandteil eines medizinischen Mittels zur Prophylaxe und Behandlung von Infektionen gegen *Pseudomonas aeruginosa*.

Bevorzugt werden in dem medizinischen Mittel zur Prophylaxe und Behandlung von Infektionen gegen *Pseudomonas aeruginosa* mehr als eines der erfindungsgemäßen DNAzyme ausgewählt aus der Gruppe Seq.ID 1-341 eingesetzt. Insbesondere wird die Kombination von mindestens zwei DNAzymen bevorzugt, die gegen unterschiedliche Zielregionen der mRNA von *algD* von *Pseudomonas aeruginosa* gerichtet sind.

Durch die Variabilität bakterieller mRNA wird der Fokus auf ein Gemisch schnellspaltender DNAzyme gelegt, um die Wahrscheinlichkeit einer Resistenzbildung zu minimieren und möglichst umfangreich alle Bakterien zu adressieren.
Das erfindungsgemäße Mittel umfasst alternativ einen pharmakologisch verträglichen Träger, Hilfsstoff und/oder Lösungsmittel. Das erfindungsgemäße Mittel wird in Form von Tropfen, Mundspray, Inhalationspulvern, Granulaten, Emulsionen, Dispersionen, Mikrokapseln, Kapseln oder Injektionslösungen hergestellt und verabreicht. Das erfindungsgemäße Mittel ist unter anderem für die Inhalation oder die intravenöse, intraperitoneale, mucokutane, orale, topikale, oder occulare Verabreichung geeignet.

Als pharmakologisch verträgliche Träger werden beispielsweise Lactose, Stärke, Sorbitol, Sucrose, Cellulose, Magnesiumstearat, Dicalciumphosphat, Calciumsulfat, Talk, Mannitol, Ethylalkohol und dergleichen eingesetzt. Puder als auch Tabletten können zu 5 bis 95% aus einem derartigen Träger bestehen.
Flüssige Formulierungen umfassen Lösungen, Suspensionen, Sprays und Emulsionen, beispielsweise Injektionslösungen auf Wasserbasis oder Wasser-Propylenglycol-Basis für parenterale Injektionen.
Kapseln werden beispielsweise aus Methylcellulose, Polyvinylalkohole, Polykationen denaturierter Gelatine oder Stärke hergestellt.

Das erfindungsgemäße Mittel umfasst alternativ weitere Hilfsstoffe die die Bioverfügbarkeit und Aktivität fördern sind:
- Kationische Moleküle wie Aminosäuren, Zucker, Lipide, anorganischen Salzen und deren Derivaten
- Kationische Polymere bestehend aus Mischungen oder reinen Monomeren wie Aminosäuren, Zuckern, Lipiden und deren Derivaten
- Kationische Poly-Zuckerderivate, wie beispielsweise Chitosan
- Kationische Poly-Proteinderivate, wie Cell-penetrating-peptides (CPP) meist kurze, überdurchschnittlich kationische Peptide wie z.B. (KFF)₃K, HIV-TAT1, RTRTRFLRRTXB, (RFF)₃XB, häufig mit viel Lysin, Arginin oder Histidin
- Kationische Copolymere, wie beispielsweise poly(L-lysine)-graft-Dextran

Insbesondere Mangan-haltige Hilfsstoffe z.B. Mangan-Cl verbessern die Bioverfügbarkeit. Bevorzugterweise wird Mangan-CL in äquimolaren Mengen dem DNAzym beigefügt.
Das erfindungsgemäße Mittel wird inhalativ z.B. mit piezoelektrischen, Düsen-, Ultraschall-Aerosolgeneratoren, Soft-Mist-Inhalern, Metered Dose Inhalern oder Dry Powder Inhalern verabreicht, d.h. die Applikation in die Lunge erfolgt durch Inhalation eines Aerosols (Suspension, Pulver) mit Hilfe eines Verneblers.
Die Art der Dosierung des erfindungsgemäßen Mittels wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden.

Das medizinische Mittel enthaltend mindestens eines der erfindungsgemäßen DNAzyme ausgewählt aus der Gruppe Seq.ID 1-341 mit oder ohne Modifikationen ist zur Prophylaxe und Behandlung von Infektionen mit *Pseudomonas aeruginosa* geeignet und wird auch zur Behandlung von CF und COPD eingesetzt.
Eine Kombination der erfindungsgemäßen DNAzyme ausgewählt aus der Gruppe Seq.ID 1-341 ist möglich und steigert die Wirksamkeit.
Ebenso ist eine Kombination zweier oder mehrerer DNAzyme mit mindestens einem weiteren medizinischen Wirkstoff wie z.B. Analgetika oder Antibiotika möglich. Weiterhin ist die Kombination mit mindestens einem antiinflammatorischen Mittel, Immunmodulator, Antiasthmatikum und/oder Bronchodilatator möglich. Ebenso ist die Kombination mit mindestens einem antiparasitären, antibakteriellen, antimykotischen und/oder antiviralen Wirkstoff möglich.
Alternativ ist die Kombination mit mindestens einem dermatologischen oder kosmetischen Wirkstoff möglich.
Besonders hervorragende Eigenschaften weisen die DNAzyme DzTHM95 und DzTHM290 auf, da sie in der Kinetik besonders schnell spalten. Bevorzugt weist das erfindungsgemäße Mittel eines dieser DNAzyme auf. Alternativ weist das erfindungsgemäße Mittel beide DNAzyme DzTHM95 und DzTHM290 auf.

### Ausführungsbeispiele

### 1. Alignment für eine möglichst optimale Zielsequenz im algD-Gen

Das mittels Bioinformatik ausgewertete Alignment ist in Figur 2 dargestellt.

Eine optimale Zielsequenz zur Bindung der erfindungsgemäßen DNAzyme wird ermittelt. Es wurde ermittelt, dass die erfindungsgemäßen DNAzyme ausgewählt aus der Gruppe Seq.ID 1-341 spezifisch an Bereichen der Zielsequenz binden und diese spalten.

Für jedes DNAzym wurde nach dem erfindungsgemäßen Verfahren eine Spaltungskinetik erstellt, um die Geschwindigkeit der Spaltung zu ermitteln und die DNAzyme herauszufinden, die am schnellsten und zuverlässigsten spalten.

### 2. RNA Expression (Synthese)

Die genutzte RNA zum erfindungsgemäßen Verfahren zur Bestimmung der Funktionskinetik der DNAzyme wurde synthetisch hergestellt, dadurch werden besonders präzise und reproduzierbare Kinetiken erhalten. Die Konzentration kann so exakt eingestellt und die Sequenz vorbestimmt werden. Es ist alternativ möglich *in vivo* generierte RNA zu verwenden, wobei hier Nachteile, wie z.B. Verunreinigungen, Einfluss auf das Messergebnis nehmen können. Mit dieser *in vivo* RNA können sehr gut beispielsweise Screenings für neue DNAzyme an neuen Targets erforscht werden.

### 3. Bereitstellung der erfindungsgemäßen DNAzyme

Die Herstellung der erfindungsgemäßen DNAzyme gemäß Seq.ID 1-341 erfolgt nach einem dem Fachmann bekannten Syntheseverfahren.

DNAzyme und RNA werden quantifiziert z.B. durch Anionenaustausch High Performance HPLC (AEX-HPLC) nach Herstellerangaben. Die Separation erfolgt z.B. durch eine DNAPac PA200 4x250 mm Säule (ThermoScientific), temperiert auf 60°C. Das Injektionsvolumen beträgt 10 µL. Es wird ein Gradientenverfahren verwendet mit einer konstanten Flussrate von 1 mL/min von Eluent B (4% TEAA, 10% ACN in Milli-Q Wasser) zu Eluent A (4% TEAA, 10% ACN, 0.2 M NaClO4 in Milli-Q Wasser).

Als HPLC System wird z.B. das LaChrom Elite System mit einem Dioden-Arraydetektor verwendet und bei einer Wellenlänge von 260 nm gemessen. Peaks werden mit der integrierten EZChrome Elite Software erfasst, ausgewertet und dargestellt.

### 4. Verfahren zur Bestimmung der Funktionskinetik zur Charakterisierung der erfindungsgemäßen DNAzyme an der Zielsequenz

Um eine einfache, schnelle und kostengünstige Alternative zur Bestimmung der enzymatischen Aktivität von DNAzymen zu finden, wurde ein neues Verfahren zur Bestimmung der Funktionskinetik entwickelt. Das erfindungsgemäße Verfahren der Bestimmung der Funktionskinetik für DNAzyme, Ribozyme und anderen auf katalytischen Nukleinsäuren basierenden Moleküle hat den Vorteil zulassungskonforme Geräte zu nutzen. Desweiteren können vergleichbare und reproduzierbare Ergebnisse unabhängig von Laboren erzielt werden, da synthetische RNA nach GMP Herstellung genutzt werden können. Nachteile, die bei der *in vivo* Biosynthese und *in vitro* Amplifikation von RNA entstehen werden behoben. Diese Nachteile können Verunreinigungen und Qualitätsmängel bei benötigten Aufreinigungsprozessen sein, Mutationen des bakteriellen Genoms oder der zu amplifizierenden RNA sein, benötigter Zugang und Infrastruktur zur Amplifikation der RNA auf *in vivo* und *in vitro* Ebene und schlechte Reproduzierbarkeit.

Bezüglich der Auswertung der Ergebnisse kann das erfindungsgemäße Verfahren durch HPLC Unterstützung objektive und zulassungskonforme Auswertungen erstellen die bei herkömmlichen Methoden derzeit in Frage zu stellen sind.

Das Verfahren beinhält eine qualitative und quantitative Analyse in welchem Ausmaß und welcher Geschwindigkeit die erfindungsgemäßen DNAzyme der Seq.ID 1-341 die Zielsequenz binden und spalten.

Das Verfahren zur Bestimmung der Funktionskinetik von DNAzymen weist folgende Schritte auf:
i) in einem Reaktionsgefäß wird mindestens ein RNA-Molekül enthaltend die Zielsequenz für das DNAzym mit einer Länge von mindestens 15 Nukleotiden mit mindestens einem DNAzym in einem Reaktionspuffer inkubiert
ii)das Reaktionsgefäß wird während der Inkubation auf mind. 20°C temperiert wird
iii) das Inkubat wird durch mindenstens drei aufeinanderfolgende Injektionen aus demselben Reaktionsgefäß auf eine stationäre Phase injiziert
iv) die Trennung des Inkubats auf der stationären Phase erfolgt mit einem Elutionsmittel durch Hochleistungsflüssigkeitschromatographie, so dass die RNA und die Spaltprodukte der RNA detektiert werden
v) Detektion der RNA, der Spaltprodukte der RNA sowie des DNAzyms erfolgt hinsichtlich Konzentrationsänderung über den Zeitverlauf

Die Bestimmung der Funktionskinetik von DNAzymen wurde zum Beispiel mit einem geeigneten jedoch temperiertem Puffer und physiologischen Salzgehalt mit jeweils 10 µM komplementärer RNA durchgeführt. Es wird eine synthetische RNA verwendet, um besonders präzise und robuste Ergebnisse zu erzielen. Über das Temperaturprofil des Puffers konnten DNAzyme ermittelt werden, welche an die speziellen Applikationsbedingungen angepasst sind.
Die Reaktion startet durch Zugabe von 2 µM des mindestens einen DNAzyms (ausgewählt aus der Gruppe Seq.ID 1-341) und wird automatisch gestoppt durch Injektion in die HPLC.
Die Kinetik wird durchgeführt durch nachfolgende Injektion vom selben Reaktionsgefäß. Der automatische Probenhalter der HPLC wird auf die gewünschte Reaktionstemperatur von mindestens 20°C temperiert. Eine Temperatur von 35°C ist für DNAzyme, die für die Applikation an der Lunge verwendet werden optimal. Die Temperatur wird in dem Probenhalter über die Reaktions- und Messzeit konstant gehalten. Eigene Experimente zeigen dass DNAzyme für die systemische Anwendung auf 37°C temperiert werden sollten und DNAzyme für die dermale Applikation auf 32°C.

Es wurde herausgefunden, dass gut auswertbare Messergebnisse erzielt werden mit 10 µM RNA und 2 µM DNAzym. Es wurde ebenfalls herausgefunden, dass als stationäre Phase Anionenaustauscher(IEC)-, Umkehrphase(RP)-, Größenausschluss(SEC)- oder Normalphasen(NP)-Säulenmaterial geeignet ist. Die stationäre Phase kann auch ein Hybrid aus mindestens einem der zuvor genannten Säulenarten sein.
Das Verfahren erfolgt alternativ durch zeitlich versetzte Abstoppung der Reaktion z.B. durch EDTA und kann mit nachträglicher Injektion auch aus mehreren Gefäßen durchgeführt werden. In einem Ausführungsbeispiel wird das Verfahren mit einem Referenzstandard aus synthetischer RNA oder DNA durchgeführt. Das Verfahren kann außer mit HPLC auch mit UHPLC, FPLC oder SEC durchgeführt werden, die Detektion erfolgt mittels UV/VIS Analyse auch mit Fluoreszenzdetektion, MS Analyse oder IR Analyse.

Der Vergleich der Funktionskinetiken aller DNAzyme gemäß Seq.ID 1-341 ergab, dass die DNAzyme DzTHM290 und DzTHM95 besonders gut, zuverlässig und sehr schnell schneiden und ein Temperaturoptimum besitzen, das zum Applikationsort Lunge optimal angepasst ist. Es wurde weiterhin herausgefunden, dass die DNAzyme DzTHM290 und DzTHM95 im Vergleich zu bekannten DNAzymen (Dz13 oder hdg40) schneller schneiden.

Das Verfahren ist nicht eingeschränkt auf die erfindungsgemäßen DNAzyme, sondern kann für beliebige katalytisch aktive Nukleinsäuremoleküle (DNAzym, Ribozyme und katalytisch-aktive Nukleinsäuren auch solche, die aus einer Galenik heraus isoliert wurden) und beliebige Zielsequenzen an RNA, DNA, synthetischer oder amplifizierter RNA oder DNA angewendet werden.
Mit Galenik oder Arzneiform ist beispielsweise eine Tablette, Salbe oder eine spezielle Formulierung in der das DNAzym verpackt ist, gemeint. Hier ist die Funktionskinetik besonders wichtig in Bezug auf Stabilitätsstudien der Arzneimittel, bei denen die DNAzyme in Verkaufsware auf Lager gelegt sind und zu bestimmten Zeitpunkten auf ihre Aktivität überprüft werden müssen. Dabei kann ein Herauslösen der DNAzyme aus der Galenik erforderlich sein.

### Abbildungslegenden

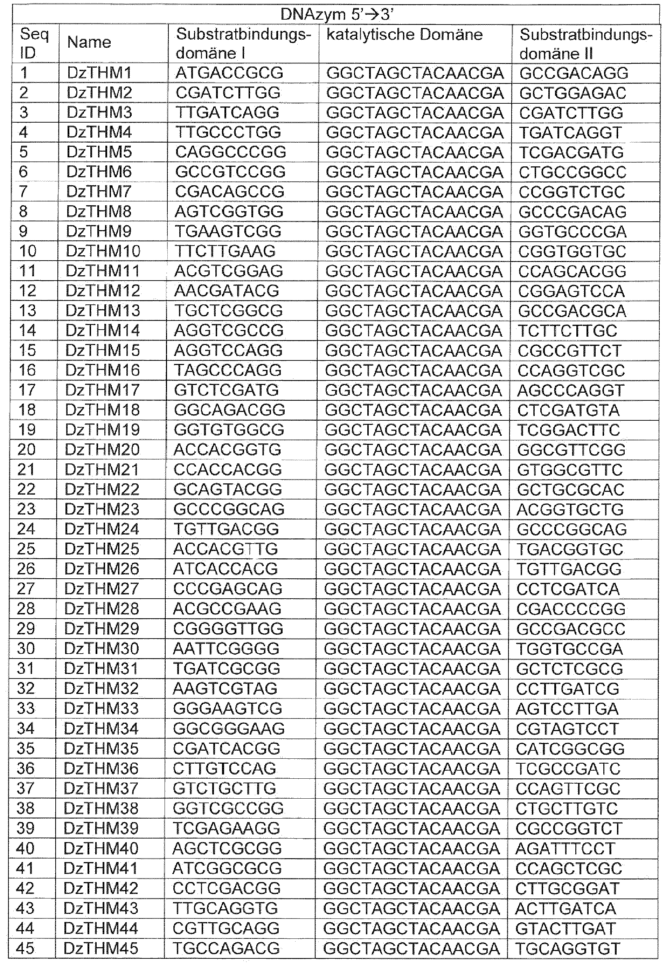

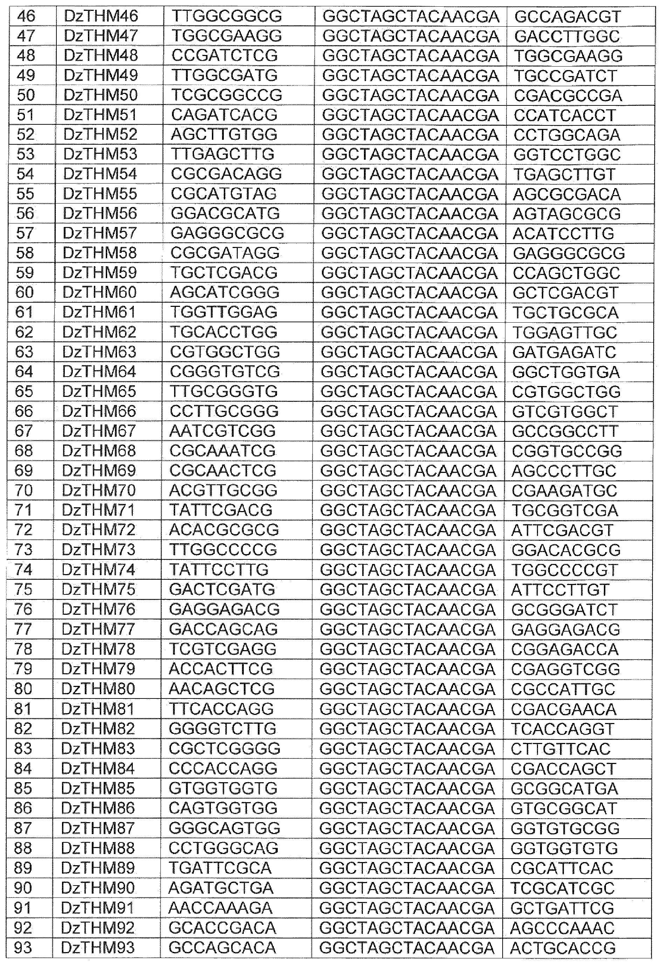

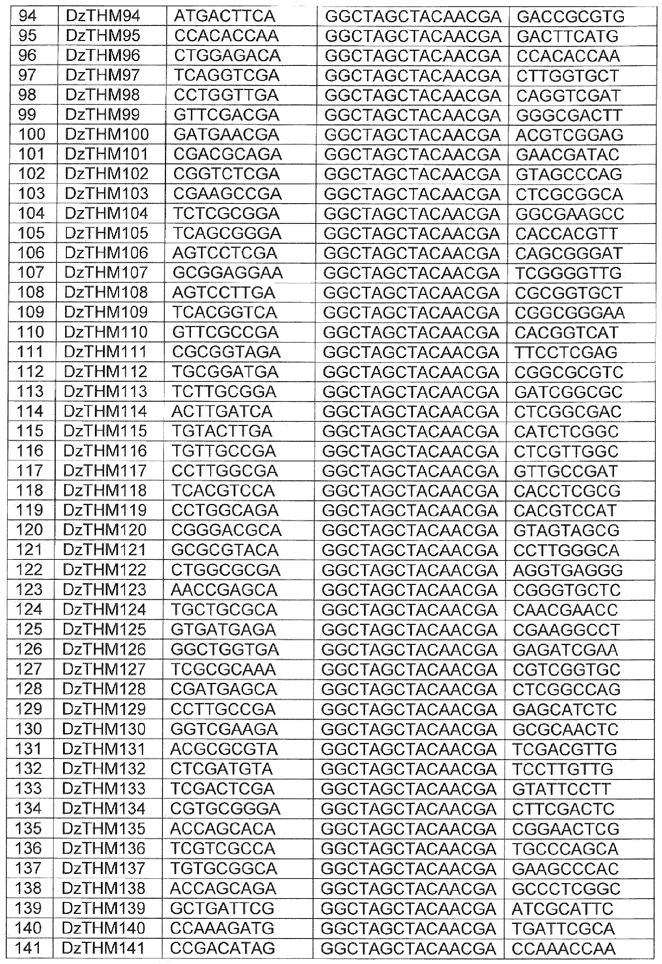

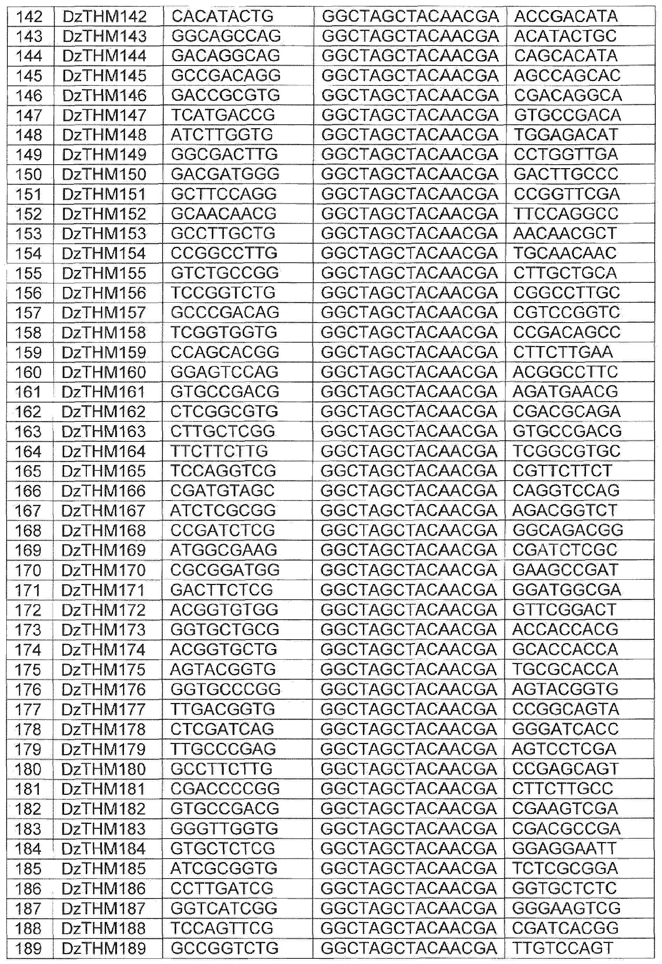

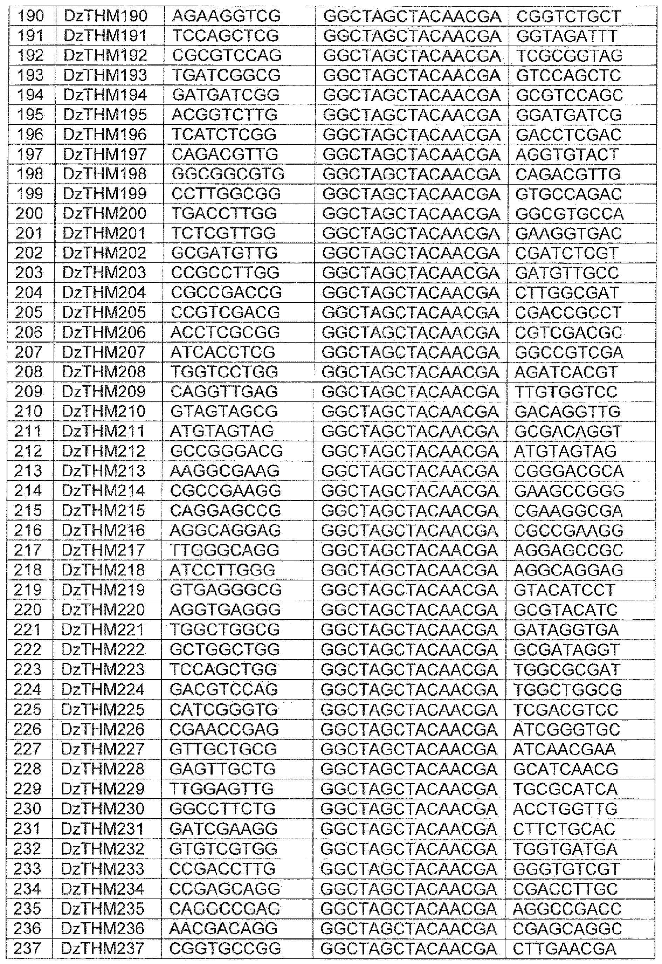

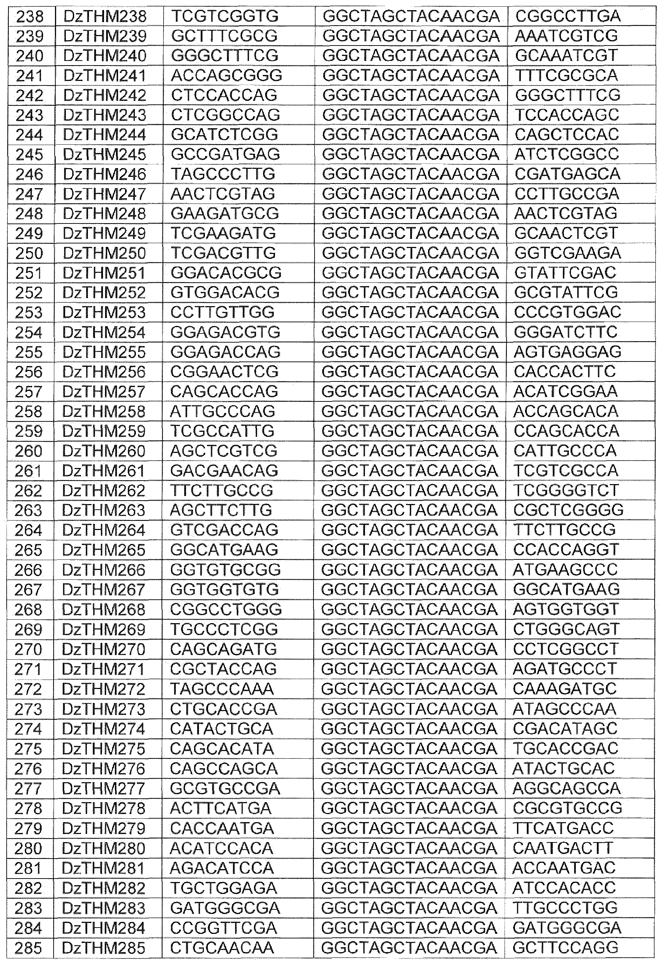

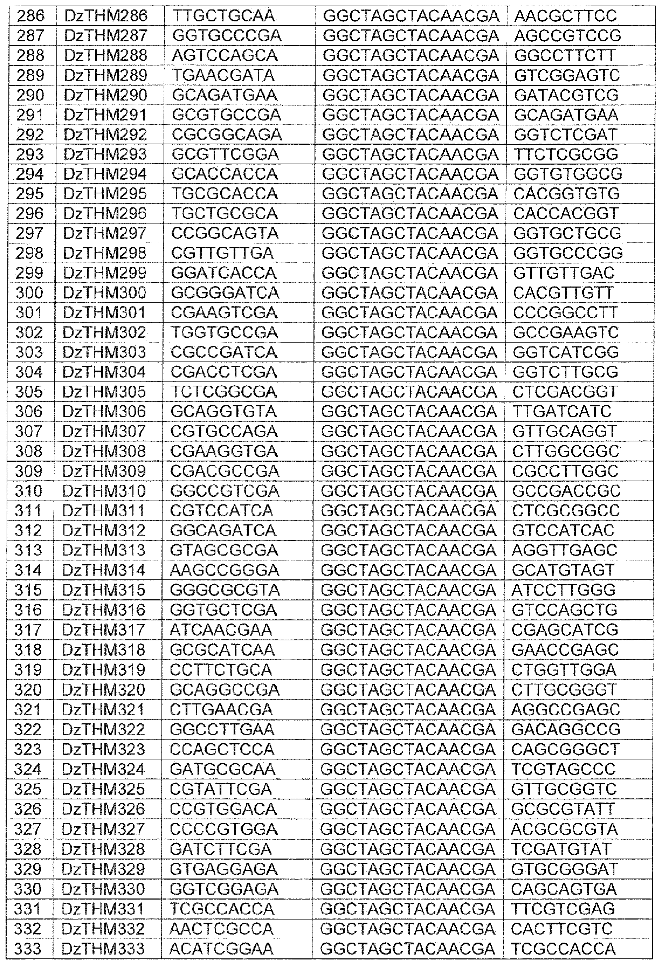

Fig. 1 zeigt die Nukleotidsequenzen der erfindungsgemäßen DNAzyme der Gruppe Seq.ID 1-341, wobei die Substratbindungsdomäne I und II, sowie die katalytische Domäne dargestellt sind.
Fig. 2 zeigt die Nukleotidsequenz des *algD*-Gens von *Pseudomonas aeruginosa* (Seq.ID 342). Dabei wurde daraus abgeleitete RNA als eine spezielle Targetregion für die Bindung der erfindungsgemäßen DNAzyme gemäß Seq.ID 1-341 verwendet. Gezeigt sind die ausgewählten Bereiche, die sich zwischen den variablen Regionen (schwarz hinterlegt) die weit genug auseinander liegen und in der sich eine Purin-Pyrimidin Schnittstelle für ein DNAzym befindet. Die Abkürzungen bedeuten: (Nukleotide A=Adenin, C=Cytosin, T=Thymin, G=Guanin, Y=Pyrimidin (C oder T), R=Purin (A oder G), S=strong (C oder G); bezogen auf die "starken" 3 Wasserstoffbrücken, die diese Basen ausbilden und K=Ketogruppe (G oder T).
   Als besonders gute Bindestelle für DNAzym DzTHM95 wurde die Bindestelle Nukleotide 70-88 in der Seq.ID 342 ausgewählt.
   Als besonders gute Bindestelle für das DNAzym DzTHM290 wurde die Bindestelle Nukleotide 231-249 in der Seq.ID 342 ausgewählt.

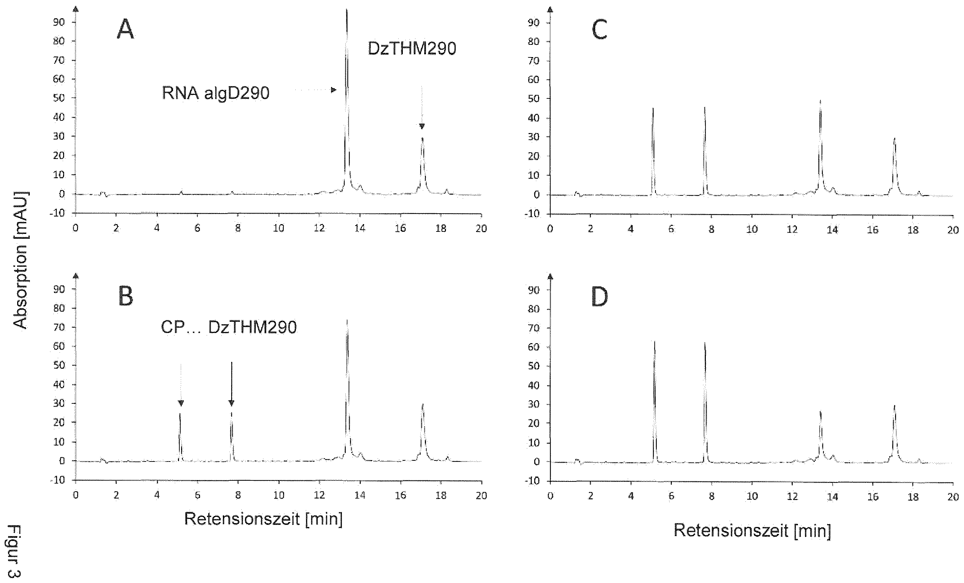

Fig. 3 zeigt die Auswertung der Funktionskinetik zur Charakterisierung der erfindungsgemäßen DNAzyme an der Zielsequenz in Form von vier Chromatogrammen (A-D) die durch aufeinanderfolgende Injektionen aus demselben Reaktionsgefäß nach HPLC-Verfahren entstanden sind. Beispielhaft werden die Reaktion des DNAzym DzTHM290 und seine Kinetik dargestellt.

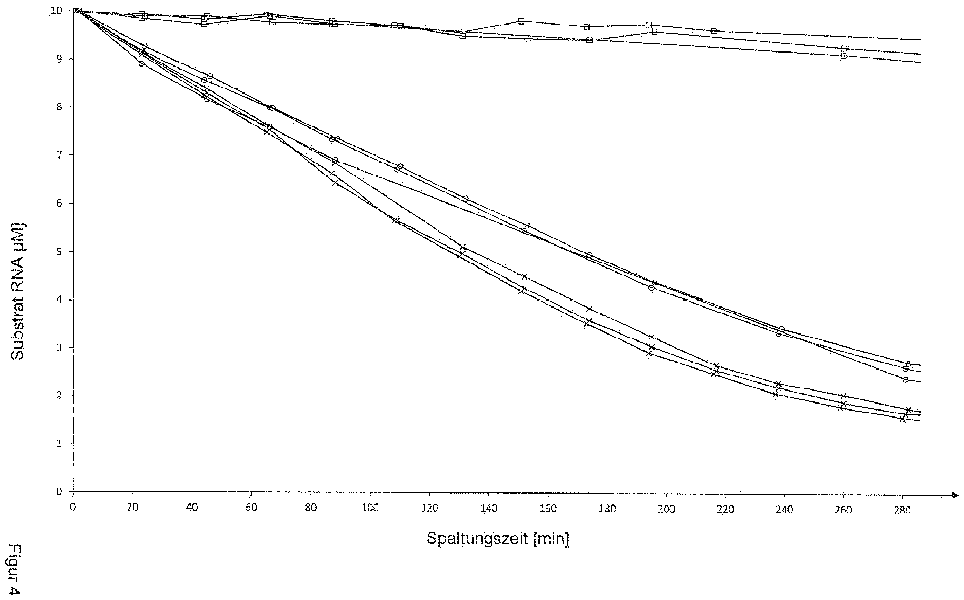

Fig. 4 zeigt den Abbau von RNA durch ein DNAzym in dreifacher Wiederholung. Exemplarisch ist die Reaktion von DzTHM290 (untere drei Linien, Datenpunkte mit Kreuzen dargestellt), DzTHM95 (mittlere drei Linien, Datenpunkte mit Kreisen dargestellt) und ein Vergleichs-DNAzym (obere drei Linien, Datenpunkte mit Quadraten dargestellt) gezeigt.

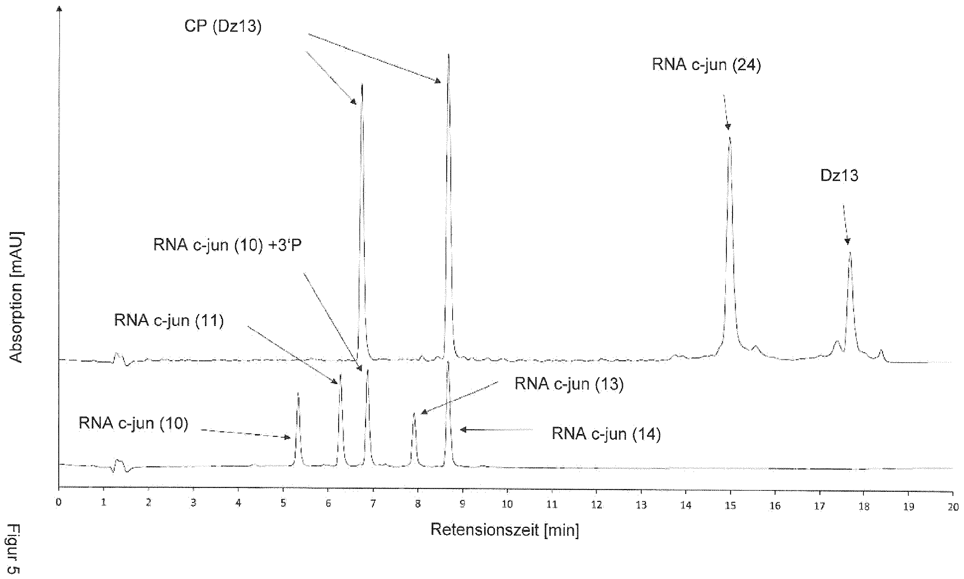

Fig. 5 zeigt die Ergebnisse der Messung zur Bestimmung der Funktionskinetik von DNAzymen, insbesondere der DNAzyme ausgewählt aus SeqID 1-341, mittels HPLC.

Zur besseren Übersicht sind in nachfolgender Tabelle die Nukleotidsequenzen der in diesem Experimente eingesetzten Moleküle (DNAzyme und RNA-Targets) aufgeführt. Die Schnittstelle der DNAzyme am jeweiligen RNA-Target kann exakt bestimmt werden:
Nachfolgend exemplarisch an Vergleichs-DNAzym Dz13 durchgeführt:
   Die Schnittstelle liegt zwischen Uridin Nr. 14 und dem zur 5'-Seite folgendem Phosphat.

Die Figur zeigt, dass besonders das Cleavage Produkt (CP) zum 3'-Ende (oberes Chromatogramm, rechter CP[Dz13] Peak) als Referenzstandard geeignet ist, da es Äquivalent zu RNA c-jun (14) ist (unteres Chromatogramm RNA c-jun(14)). Hingegen ist das 5'-Produkt (oberes Chromatogramm, linker CP[Dz13] Peak) mit einem intramolekularem Phosphatester versehen, der die Retentionszeit zu einem normal gebundenen Phosphat (unteres Chromatogramm RNA c-jun(10)+P) leicht verändert. Daher eignet sich das Cleavage Produkt (CP) zur 3'-Seite (oberes Chromatogramm, CP[Dz13] rechter Peak) als Standard, da es 100% identisch als synthetischer Standard zur Verfügung gestellt werden kann.

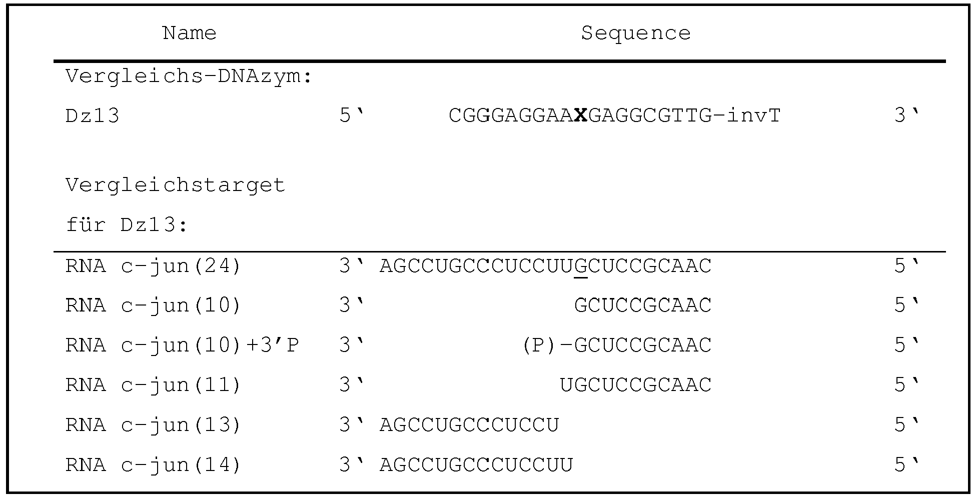

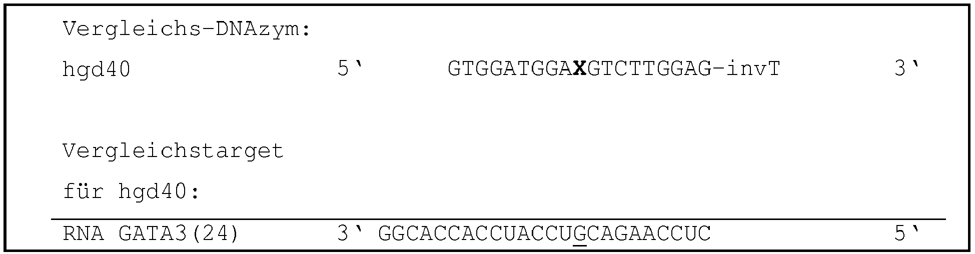

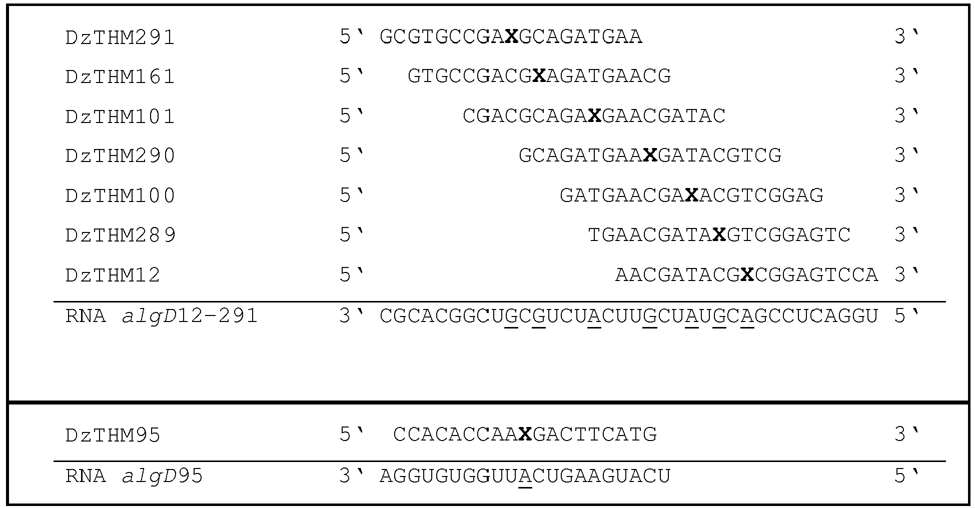

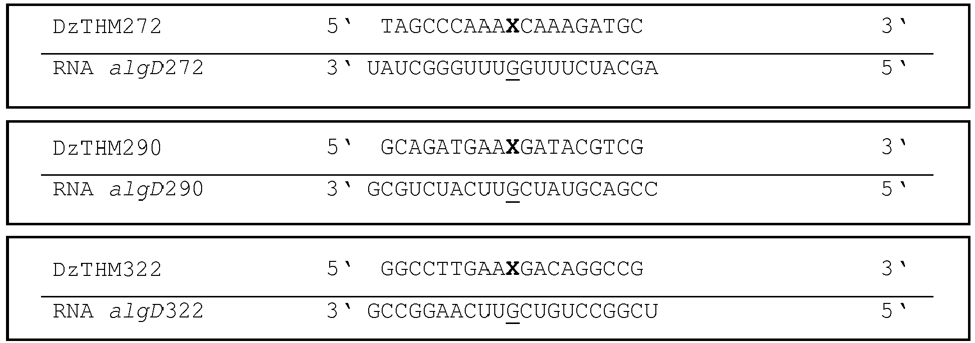

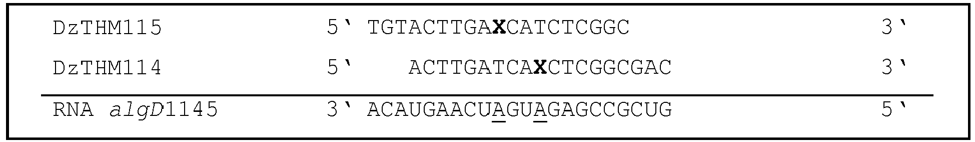

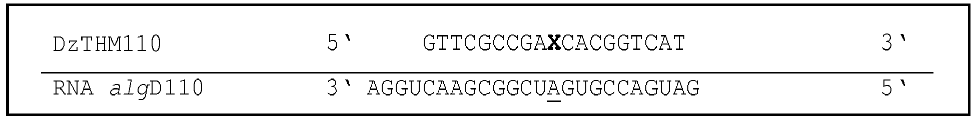

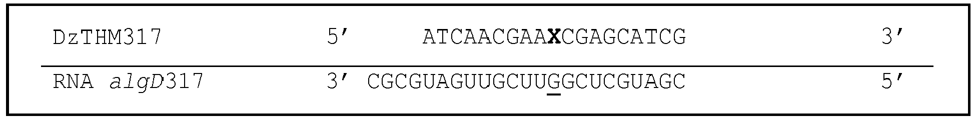

Bei Dz13 wurde die katalytische Domäne, also das 10-23 Motiv "GGCTAGCTACAACGA" durch ein "X" ersetzt.

Die Spaltungsstellen liegen zwischen dem 3'-Phosphat des unterstrichenen Nukleotides und dem 3'-Nachbarnukleosid.

3'-Ende und 5'-Ende sind nicht phosphoryliert. Das invT steht für die Modifikation mit einem invertem Thymidin.

## Patentansprüche

1. Mittel zur Prophylaxe und Behandlung von Infektionen mit *Pseudomonas aeruginosa* **dadurch gekennzeichnet dass** es mindestens ein DNAzym ausgewählt aus Seq.ID 1-341 aufweist, wobei dieses eine zentrale katalytische Domäne sowie eine Substratbindungsdomäne I und Substratbindungsdomäne II aufweist, die komplementär zu einer Zielsequenz der mRNA von *Pseudomonas aeruginosa* ist.

2. Mittel gemäß Anspruch 1 **dadurch gekennzeichnet dass** diese Zielsequenz die Sequenz gemäß Seq.ID 342 ist.

3. Mittel gemäß Anspruch 1 **dadurch gekennzeichnet dass** mindestens eines der Nukleotide der Substratbindungsdomäne I und/oder II eine Modifikation aufweist.

4. Mittel gemäß einem der vorangegangenen Ansprüche **dadurch gekennzeichnet dass** mindestens eines der Nukleotide der katalytischen Domäne eine Modifikation aufweist.

5. Mittel gemäß einem der vorangegangenen Ansprüche **dadurch gekennzeichnet dass** die Substratbindungsdomäne I oder II zumindest zu 90% komplementär zu einer Zielsequenz der mRNA von *Pseudomonas aeruginosa* ist.

6. Mittel gemäß einem der vorangegangenen Ansprüche **dadurch gekennzeichnet dass** die Sequenz des DNAzym gemäß Seq.ID 1-341 mindestens 8 Nukleotiden für die Substratbindungsdomäne I und mindestens 8 Nukleotide für Substratbindungsdomäne II umfasst.

7. Mittel gemäß einem der vorangegangenen Ansprüche **dadurch gekennzeichnet dass** das mindestens eine DNAzym ausgewählt aus Seq.ID 1-341 kombiniert werden kann mit mindestens einem antiinflammatorischen Mittel, Immunmodulator, Antiasthmatikum, Analgetikum, Antipyretikum und/oder Bronchodilatator.

8. Mittel gemäß einem der vorangegangenen Ansprüche **dadurch gekennzeichnet, dass** das mindestens eine DNAzym kombiniert werden kann mit mindestens einem antiparasitären, antibakteriellen, antimykotischen und/oder antiviralen Wirkstoff.

9. Mittel gemäß einem der vorangegangenen Ansprüche **dadurch gekennzeichnet dass** es zur Prophylaxe und Behandlung von Cystischer Fibrose und/oder COPD geeignet ist.

10. Mittel gemäß einem der vorangegangenen Ansprüche **dadurch gekennzeichnet dass** das Mittel als Teil einer pharmazeutisch akzeptablen Komposition inhalativ, als Spray, Tropfen, oral oder in Tablettenform verabreicht wird

11. Mittel gemäß einem der vorangegangenen Ansprüche **dadurch gekennzeichnet dass** das mindestens eine DNAzym ausgewählt aus Seq.ID 1-341 DzTHM 95 oder DzTHM 290 ist.

12. Mittel gemäß einem der vorangegangenen Ansprüche **dadurch gekennzeichnet dass** es die DNAzyme DzTHM 95 und DzTHM 290 umfasst.

13. DNAzym ausgewählt aus Seq.ID 1-341, **dadurch gekennzeichnet dass** es eine katalytische Domäne, sowie zwei Substratbindedomänen I und II umfasst und komplementär zu einer Zielsequenz der mRNA von *Pseudomonas aeruginosa* gemäß Seq.ID 342 ist, wobei das DNAzym die Seq.ID 290 oder Seq.ID 95 aufweist.

14. Verfahren zur Bestimmung der Funktionskinetik von DNAzymen **dadurch gekennzeichnet dass**
i) in einem Reaktionsgefäß mindestens ein RNA-Molekül enthaltend die Zielsequenz für das DNAzym mit einer Länge von mindestens 15 Nukleotiden mit mindestens einem DNAzym in einem Reaktionspuffer inkubiert wird
ii) das Reaktionsgefäß während der Inkubation auf mind. 20 °C temperiert wird
iii) das Inkubat durch mindestens drei aufeinanderfolgende Injektionen aus demselben Reaktionsgefäß auf eine stationäre Phase injiziert wird
iv) die Trennung des Inkubats auf der stationären Phase mit einem Elutionsmittel durch Hochleistungsflüssigkeitschromatographie erfolgt, so dass die RNA und die Spaltprodukte der RNA detektiert werden
v) die Detektion der RNA, der Spaltprodukte der RNA sowie des DNAzyms hinsichtlich Konzentrationsänderung über den Zeitverlauf erfolgt

15. Verfahren gemäß Anspruch 14 **dadurch gekennzeichnet dass** die stationäre Phase Anionenaustauscher (IEC)-, Umkehrphase (RP)-, Größenausschluss (SEC)- oder Normalphasen (NP)-Säulenmaterial aufweist.


SEQUENCE LISTING

<110> Technische Hochschule Mittelhessen

<120> Erfindung betreffend Wirkstoff gegen Pseudomonas aeruginosa

<130> EP

<160> 342

<170> BiSSAP 1.3.5

<210> 1
<211> 33
<212> DNA
<213> Artificial Sequence

<223> "DNAzym "

<220>
<223> DNAzym

<400> 1
atgaccgcgg gctagctaca acgagccgac agg 33

<210> 2
<211> 33
<212> DNA
<213> Artificial Sequence

<223> "DNAzym"

<220>
<223> DNAzym

<400> 2
cgatcttggg gctagctaca acgagctgga gac 33

<210> 3
<211> 33
<212> DNA
<213> Artificial Sequence

<223> "DNAzym"

<220>
<223> DNAzym

<400> 3
ttgatcaggg gctagctaca acgacgatct tgg                                    33

<210> 4
<211> 33
<212> DNA
<213> Artificial Sequence

<223> "DNAzym"

<220>
<223> DNAzym

<400> 4
ttgccctggg gctagctaca acgatgatca ggt                                    33

<210> 5
<211> 33
<212> DNA
<213> Artificial Sequence

<223> "DNAzym"

<220>
<223> DNAzym

<400> 5
caggcccggg gctagctaca acgatcgacg atg                                    33

<210> 6
<211> 33
<212> DNA
<213> Artificial Sequence

<223> "DNAzym"

<220>
<223> DNAzym

<400> 6
gccgtccggg gctagctaca acgactgccg gcc                                    33

<210> 7
<211> 33
<212> DNA

<213> Artificial Sequence

<223> "DNAzym"

<220>
<223> DNAzym

<400> 7
cgacagccgg gctagctaca acgaccggtc tgc                                    33


<210> 8
<211> 33
<212> DNA
<213> Artificial Sequence

<223> "DNAzym"

<220>
<223> DNAzym

<400> 8
agtcggtggg gctagctaca acgagcccga cag                                    33


<210> 9
<211> 33
<212> DNA
<213> Artificial Sequence

<223> "DNAzym"

<220>
<223> DNAzym

<400> 9
tgaagtcggg gctagctaca acgaggtgcc cga                                    33


<210> 10
<211> 33
<212> DNA
<213> Artificial Sequence

<223> "DNAzym"

<220>
<223> DNAzym

<400> 10

ttcttgaagg gctagctaca acgacggtgg tgc 33

<210> 11
<211> 33
<212> DNA
<213> Artificial Sequence

<223> "DNAzym"

<220>
<223> DNAzym

<400> 11
acgtcggagg gctagctaca acgaccagca cgg 33

<210> 12
<211> 33
<212> DNA
<213> Artificial Sequence

<223> "DNAzym"

<220>
<223> DNAzym

<400> 12
aacgatacgg gctagctaca acgacggagt cca 33

<210> 13
<211> 33
<212> DNA
<213> Artificial Sequence

<223> "DNAzym"

<220>
<223> DNAzym

<400> 13
tgctcggcgg gctagctaca acgagccgac gca 33

<210> 14
<211> 33
<212> DNA
<213> Artificial Sequence

<223> "DNAzym"

<220>
<223> DNAzym

<400> 14
aggtcgccgg gctagctaca acgatcttct tgc                                    33

<210> 15
<211> 33
<212> DNA
<213> Artificial Sequence

<223> "DNAzym"

<220>
<223> DNAzym

<400> 15
aggtccaggg gctagctaca acgacgccgt tct                                    33

<210> 16
<211> 33
<212> DNA
<213> Artificial Sequence

<223> "DNAzym"

<220>
<223> DNAzym

<400> 16
tagcccaggg gctagctaca acgaccaggt cgc                                    33

<210> 17
<211> 33
<212> DNA
<213> Artificial Sequence

<223> "DNAzym"

<220>
<223> DNAzym

<400> 17
gtctcgatgg gctagctaca acgaagccca ggt                                    33

<210> 18
<211> 33
<212> DNA
<213> Artificial Sequence

<223> "DNAzym"

<220>
<223> DNAzym

<400> 18
ggcagacggg gctagctaca acgactcgat gta                                    33



<210> 19
<211> 33
<212> DNA
<213> Artificial Sequence

<223> "DNAzym"

<220>
<223> DNAzym

<400> 19
ggtgtggcgg gctagctaca acgatcggac ttc                                    33



<210> 20
<211> 33
<212> DNA
<213> Artificial Sequence

<223> "DNAzym"

<220>
<223> DNAzym

<400> 20
accacggtgg gctagctaca acgaggcgtt cgg                                    33



<210> 21
<211> 33
<212> DNA
<213> Artificial Sequence

<223> "DNAzym"

<220>
<223> DNAzym

<400> 21
ccaccacggg gctagctaca acgagtggcg ttc                                    33

<210> 22
<211> 33
<212> DNA
<213> Artificial Sequence

<223> "DNAzym"

<220>
<223> DNAzym

<400> 22
gcagtacggg gctagctaca acgagctgcg cac                                    33

<210> 23
<211> 33
<212> DNA
<213> Artificial Sequence

<223> "DNAzym"

<220>
<223> DNAzym

<400> 23
gcccggcagg gctagctaca acgaacggtg ctg                                    33

<210> 24
<211> 33
<212> DNA
<213> Artificial Sequence

<223> "DNAzym"

<220>
<223> DNAzym

<400> 24
tgttgacggg gctagctaca acgagcccgg cag                                    33

<210> 25

<211> 33
<212> DNA
<213> Artificial Sequence

<223> "DNAzym"

<220>
<223> DNAzym

<400> 25
accacgttgg gctagctaca acgatgacgg tgc                                    33

<210> 26
<211> 33
<212> DNA
<213> Artificial Sequence

<223> "DNAzym"

<220>
<223> DNAzym

<400> 26
atcaccacgg gctagctaca acgatgttga cgg                                    33

<210> 27
<211> 33
<212> DNA
<213> Artificial Sequence

<223> "DNAzym"

<220>
<223> DNAzym

<400> 27
cccgagcagg gctagctaca acgacctcga tca                                    33

<210> 28
<211> 33
<212> DNA
<213> Artificial Sequence

<223> "DNAzym"

<220>
<223> DNAzym

<400> 28
acgccgaagg gctagctaca acgacgaccc cgg                                    33

<210> 29
<211> 33
<212> DNA
<213> Artificial Sequence

<223> "DNAzym"

<220>
<223> DNAzym

<400> 29
cggggttggg gctagctaca acgagccgac gcc                                    33

<210> 30
<211> 33
<212> DNA
<213> Artificial Sequence

<223> "DNAzym"

<220>
<223> DNAzym

<400> 30
aattcggggg gctagctaca acgatggtgc cga                                    33

<210> 31
<211> 33
<212> DNA
<213> Artificial Sequence

<223> "DNAzym"

<220>
<223> DNAzym

<400> 31
tgatcgcggg gctagctaca acgagctctc gcg                                    33

<210> 32
<211> 33
<212> DNA

<213> Artificial Sequence

<223> "DNAzym"

<220>
<223> DNAzym

<400> 32
aagtcgtagg gctagctaca acgaccttga tcg                                    33

<210> 33
<211> 33
<212> DNA
<213> Artificial Sequence

<223> "DNAzym"

<220>
<223> DNAzym

<400> 33
gggaagtcgg gctagctaca acgaagtcct tga                                    33

<210> 34
<211> 33
<212> DNA
<213> Artificial Sequence

<223> "DNAzym"

<220>
<223> DNAzym

<400> 34
ggcgggaagg gctagctaca acgacgtagt cct                                    33

<210> 35
<211> 33
<212> DNA
<213> Artificial Sequence

<223> "DNAzym"

<220>
<223> DNAzym

<400> 35

cgatcacggg gctagctaca acgacatcgg cgg                                    33

<210> 36
<211> 33
<212> DNA
<213> Artificial Sequence

<223> "DNAzym"

<220>
<223> DNAzym

<400> 36
cttgtccagg gctagctaca acgatcgccg atc                                    33

<210> 37
<211> 33
<212> DNA
<213> Artificial Sequence

<223> "DNAzym"

<220>
<223> DNAzym

<400> 37
gtctgcttgg gctagctaca acgaccagtt cgc                                    33

<210> 38
<211> 33
<212> DNA
<213> Artificial Sequence

<223> "DNAzym"

<220>
<223> DNAzym

<400> 38
ggtcgccggg gctagctaca acgactgctt gtc                                    33

<210> 39
<211> 33
<212> DNA
<213> Artificial Sequence

<223> "DNAzym"

<220>
<223> DNAzym

<400> 39
tcgagaaggg gctagctaca acgacgccgg tct                                    33

<210> 40
<211> 33
<212> DNA
<213> Artificial Sequence

<223> "DNAzym"

<220>
<223> DNAzym

<400> 40
agctcgcggg gctagctaca acgaagattt cct                                    33

<210> 41
<211> 33
<212> DNA
<213> Artificial Sequence

<223> "DNAzym"

<220>
<223> DNAzym

<400> 41
atcggcgcgg gctagctaca acgaccagct cgc                                    33

<210> 42
<211> 33
<212> DNA
<213> Artificial Sequence

<223> "DNAzym"

<220>
<223> DNAzym

<400> 42
cctcgacggg gctagctaca acgacttgcg gat                                    33

<210> 43
<211> 33
<212> DNA
<213> Artificial Sequence

<223> "DNAzym"

<220>
<223> DNAzym

<400> 43
ttgcaggtgg gctagctaca acgaacttga tca                                    33



<210> 44
<211> 33
<212> DNA
<213> Artificial Sequence

<223> "DNAzym"

<220>
<223> DNAzym

<400> 44
cgttgcaggg gctagctaca acgagtactt gat                                    33



<210> 45
<211> 33
<212> DNA
<213> Artificial Sequence

<223> "DNAzym"

<220>
<223> DNAzym

<400> 45
tgccagacgg gctagctaca acgatgcagg tgt                                    33



<210> 46
<211> 33
<212> DNA
<213> Artificial Sequence

<223> "DNAzym"

<220>
<223> DNAzym

<400> 46
ttggcggcgg gctagctaca acgagccaga cgt                                    33

<210> 47
<211> 33
<212> DNA
<213> Artificial Sequence

<223> "DNAzym"

<220>
<223> DNAzym

<400> 47
tggcgaaggg gctagctaca acgagacctt ggc                                    33

<210> 48
<211> 33
<212> DNA
<213> Artificial Sequence

<223> "DNAzym"

<220>
<223> DNAzym

<400> 48
ccgatctcgg gctagctaca acgatggcga agg                                    33

<210> 49
<211> 33
<212> DNA
<213> Artificial Sequence

<223> "DNAzym"

<220>
<223> DNAzym

<400> 49
ttggcgatgg gctagctaca acgatgccga tct                                    33

<210> 50

<211> 33
<212> DNA
<213> Artificial Sequence

<223> "DNAzym"

<220>
<223> DNAzym

<400> 50
tcgcggccgg gctagctaca acgacgacgc cga                                    33


<210> 51
<211> 33
<212> DNA
<213> Artificial Sequence

<223> "DNAzym"

<220>
<223> DNAzym

<400> 51
cagatcacgg gctagctaca acgaccatca cct                                    33


<210> 52
<211> 33
<212> DNA
<213> Artificial Sequence

<223> "DNAzym"

<220>
<223> DNAzym

<400> 52
agcttgtggg gctagctaca acgacctggc aga                                    33


<210> 53
<211> 33
<212> DNA
<213> Artificial Sequence

<223> "DNAzym"

<220>
<223> DNAzym

<400> 53
ttgagcttgg gctagctaca acgaggtcct ggc                                    33

<210> 54
<211> 33
<212> DNA
<213> Artificial Sequence

<223> "DNAzym"

<220>
<223> DNAzym

<400> 54
cgcgacaggg gctagctaca acgatgagct tgt                                    33

<210> 55
<211> 33
<212> DNA
<213> Artificial Sequence

<223> "DNAzym"

<220>
<223> DNAzym

<400> 55
cgcatgtagg gctagctaca acgaagcgcg aca                                    33

<210> 56
<211> 33
<212> DNA
<213> Artificial Sequence

<223> "DNAzym"

<220>
<223> DNAzym

<400> 56
ggacgcatgg gctagctaca acgaagtagc gcg                                    33

<210> 57
<211> 33
<212> DNA

<213> Artificial Sequence

<223> "DNAzym"

<220>
<223> DNAzym

<400> 57
gagggcgcgg gctagctaca acgaacatcc ttg                                    33

<210> 58
<211> 33
<212> DNA
<213> Artificial Sequence

<223> "DNAzym"

<220>
<223> DNAzym

<400> 58
cgcgataggg gctagctaca acgagagggc gcg                                    33

<210> 59
<211> 33
<212> DNA
<213> Artificial Sequence

<223> "DNAzym"

<220>
<223> DNAzym

<400> 59
tgctcgacgg gctagctaca acgaccagct ggc                                    33

<210> 60
<211> 33
<212> DNA
<213> Artificial Sequence

<223> "DNAzym"

<220>
<223> DNAzym

<400> 60

agcatcgggg gctagctaca acgagctcga cgt                                    33

<210> 61
<211> 33
<212> DNA
<213> Artificial Sequence

<223> "DNAzym"

<220>
<223> DNAzym

<400> 61
tggttggagg gctagctaca acgatgctgc gca                                    33

<210> 62
<211> 33
<212> DNA
<213> Artificial Sequence

<223> "DNAzym"

<220>
<223> DNAzym

<400> 62
tgcacctggg gctagctaca acgatggagt tgc                                    33

<210> 63
<211> 33
<212> DNA
<213> Artificial Sequence

<223> "DNAzym"

<220>
<223> DNAzym

<400> 63
cgtggctggg gctagctaca acgagatgag atc                                    33

<210> 64
<211> 33
<212> DNA
<213> Artificial Sequence

<223> "DNAzym"

<220>
<223> DNAzym

<400> 64
cgggtgtcgg gctagctaca acgaggctgg tga 33

<210> 65
<211> 33
<212> DNA
<213> Artificial Sequence

<223> "DNAzym"

<220>
<223> DNAzym

<400> 65
ttgcgggtgg gctagctaca acgacgtggc tgg 33

<210> 66
<211> 33
<212> DNA
<213> Artificial Sequence

<223> "DNAzym"

<220>
<223> DNAzym

<400> 66
ccttgcgggg gctagctaca acgagtcgtg gct 33

<210> 67
<211> 33
<212> DNA
<213> Artificial Sequence

<223> "DNAzym"

<220>
<223> DNAzym

<400> 67
aatcgtcggg gctagctaca acgagccggc ctt 33

<210> 68
<211> 33
<212> DNA
<213> Artificial Sequence

<223> "DNAzym"

<220>
<223> DNAzym

<400> 68
cgcaaatcgg gctagctaca acgacggtgc cgg                                    33



<210> 69
<211> 33
<212> DNA
<213> Artificial Sequence

<223> "DNAzym"

<220>
<223> DNAzym

<400> 69
cgcaactcgg gctagctaca acgaagccct tgc                                    33



<210> 70
<211> 33
<212> DNA
<213> Artificial Sequence

<223> "DNAzym"

<220>
<223> DNAzym

<400> 70
acgttgcggg gctagctaca acgacgaaga tgc                                    33



<210> 71
<211> 33
<212> DNA
<213> Artificial Sequence

<223> "DNAzym"

<220>
<223> DNAzym

<400> 71
tattcgacgg gctagctaca acgatgcggt cga                                33

<210> 72
<211> 33
<212> DNA
<213> Artificial Sequence

<223> "DNAzym"

<220>
<223> DNAzym

<400> 72
acacgcgcgg gctagctaca acgaattcga cgt                                33

<210> 73
<211> 33
<212> DNA
<213> Artificial Sequence

<223> "DNAzym"

<220>
<223> DNAzym

<400> 73
ttggccccgg gctagctaca acgaggacac gcg                                33

<210> 74
<211> 33
<212> DNA
<213> Artificial Sequence

<223> "DNAzym"

<220>
<223> DNAzym

<400> 74
tattccttgg gctagctaca acgatggccc cgt                                33

<210> 75

<211> 33
<212> DNA
<213> Artificial Sequence

<223> "DNAzym"

<220>
<223> DNAzym

<400> 75
gactcgatgg gctagctaca acgaattcct tgt                                    33

<210> 76
<211> 33
<212> DNA
<213> Artificial Sequence

<223> "DNAzym"

<220>
<223> DNAzym

<400> 76
gaggagacgg gctagctaca acgagcggga tct                                    33

<210> 77
<211> 33
<212> DNA
<213> Artificial Sequence

<223> "DNAzym"

<220>
<223> DNAzym

<400> 77
gaccagcagg gctagctaca acgagaggag acg                                    33

<210> 78
<211> 33
<212> DNA
<213> Artificial Sequence

<223> "DNAzym"

<220>
<223> DNAzym

<400> 78
tcgtcgaggg gctagctaca acgacggaga cca                                    33

<210> 79
<211> 33
<212> DNA
<213> Artificial Sequence

<223> "DNAzym"

<220>
<223> DNAzym

<400> 79
accacttcgg gctagctaca acgacgaggt cgg                                    33

<210> 80
<211> 33
<212> DNA
<213> Artificial Sequence

<223> "DNAzym"

<220>
<223> DNAzym

<400> 80
aacagctcgg gctagctaca acgacgccat tgc                                    33

<210> 81
<211> 33
<212> DNA
<213> Artificial Sequence

<223> "DNAzym"

<220>
<223> DNAzym

<400> 81
ttcaccaggg gctagctaca acgacgacga aca                                    33

<210> 82
<211> 33
<212> DNA

<213> Artificial Sequence

<223> "DNAzym"

<220>
<223> DNAzym

<400> 82
ggggtcttgg gctagctaca acgatcacca ggt                                    33

<210> 83
<211> 33
<212> DNA
<213> Artificial Sequence

<223> "DNAzym"

<220>
<223> DNAzym

<400> 83
cgctcggggg gctagctaca acgacttgtt cac                                    33

<210> 84
<211> 33
<212> DNA
<213> Artificial Sequence

<223> "DNAzym"

<220>
<223> DNAzym

<400> 84
cccaccaggg gctagctaca acgacgacca gct                                    33

<210> 85
<211> 33
<212> DNA
<213> Artificial Sequence

<223> "DNAzym"

<220>
<223> DNAzym

<400> 85

gtggtggtgg gctagctaca acgagcggca tga                                    33

<210> 86
<211> 33
<212> DNA
<213> Artificial Sequence

<223> "DNAzym"

<220>
<223> DNAzym

<400> 86
cagtggtggg gctagctaca acgagtgcgg cat                                    33

<210> 87
<211> 33
<212> DNA
<213> Artificial Sequence

<223> "DNAzym"

<220>
<223> DNAzym

<400> 87
gggcagtggg gctagctaca acgaggtgtg cgg                                    33

<210> 88
<211> 33
<212> DNA
<213> Artificial Sequence

<223> "DNAzym"

<220>
<223> DNAzym

<400> 88
cctgggcagg gctagctaca acgaggtggt gtg                                    33

<210> 89
<211> 33
<212> DNA
<213> Artificial Sequence

<223> "DNAzym"

<220>
<223> DNAzym

<400> 89
tgattcgcag gctagctaca acgacgcatt cac                                    33

<210> 90
<211> 33
<212> DNA
<213> Artificial Sequence

<223> "DNAzym"

<220>
<223> DNAzym

<400> 90
agatgctgag gctagctaca acgatcgcat cgc                                    33

<210> 91
<211> 33
<212> DNA
<213> Artificial Sequence

<223> "DNAzym"

<220>
<223> DNAzym

<400> 91
aaccaaagag gctagctaca acgagctgat tcg                                    33

<210> 92
<211> 33
<212> DNA
<213> Artificial Sequence

<223> "DNAzym"

<220>
<223> DNAzym

<400> 92
gcaccgacag gctagctaca acgaagccca aac                                    33

<210> 93
<211> 33
<212> DNA
<213> Artificial Sequence

<223> "DNAzym"

<220>
<223> DNAzym

<400> 93
gccagcacag gctagctaca acgaactgca ccg                                    33

<210> 94
<211> 33
<212> DNA
<213> Artificial Sequence

<223> "DNAzym"

<220>
<223> DNAzym

<400> 94
atgacttcag gctagctaca acgagaccgc gtg                                    33

<210> 95
<211> 33
<212> DNA
<213> Artificial Sequence

<223> "DNAzym"

<220>
<223> DNAzym

<400> 95
ccacaccaag gctagctaca acgagacttc atg                                    33

<210> 96
<211> 33
<212> DNA
<213> Artificial Sequence

<223> "DNAzym"

<220>
<223> DNAzym

<400> 96
ctggagacag gctagctaca acgaccacac caa                                    33

<210> 97
<211> 33
<212> DNA
<213> Artificial Sequence

<223> "DNAzym"

<220>
<223> DNAzym

<400> 97
tcaggtcgag gctagctaca acgacttggt gct                                    33

<210> 98
<211> 33
<212> DNA
<213> Artificial Sequence

<223> "DNAzym"

<220>
<223> DNAzym

<400> 98
cctggttgag gctagctaca acgacaggtc gat                                    33

<210> 99
<211> 33
<212> DNA
<213> Artificial Sequence

<223> "DNAzym"

<220>
<223> DNAzym

<400> 99
gttcgacgag gctagctaca acgagggcga ctt                                    33

<210> 100

<211> 33
<212> DNA
<213> Artificial Sequence

<223> "DNAzym"

<220>
<223> DNAzym

<400> 100
gatgaacgag gctagctaca acgaacgtcg gag                                    33


<210> 101
<211> 33
<212> DNA
<213> Artificial Sequence

<223> "DNAzym"

<220>
<223> DNAzym

<400> 101
cgacgcagag gctagctaca acgagaacga tac                                    33


<210> 102
<211> 33
<212> DNA
<213> Artificial Sequence

<223> "DNAzym"

<220>
<223> DNAzym

<400> 102
cggtctcgag gctagctaca acgagtagcc cag                                    33


<210> 103
<211> 33
<212> DNA
<213> Artificial Sequence

<223> "DNAzym"

<220>
<223> DNAzym

<400> 103
cgaagccgag gctagctaca acgactcgcg gca                                    33

<210> 104
<211> 33
<212> DNA
<213> Artificial Sequence

<223> "DNAzym"

<220>
<223> DNAzym

<400> 104
tctcgcggag gctagctaca acgaggcgaa gcc                                    33

<210> 105
<211> 33
<212> DNA
<213> Artificial Sequence

<223> "DNAzym"

<220>
<223> DNAzym

<400> 105
tcagcgggag gctagctaca acgacaccac gtt                                    33

<210> 106
<211> 33
<212> DNA
<213> Artificial Sequence

<223> "DNAzym"

<220>
<223> DNAzym

<400> 106
agtcctcgag gctagctaca acgacagcgg gat                                    33

<210> 107
<211> 33
<212> DNA

<213> Artificial Sequence

<223> "DNAzym"

<220>
<223> DNAzym

<400> 107
gcggaggaag gctagctaca acgatcgggg ttg                                    33

<210> 108
<211> 33
<212> DNA
<213> Artificial Sequence

<223> "DNAzym"

<220>
<223> DNAzym

<400> 108
agtccttgag gctagctaca acgacgcggt gct                                    33

<210> 109
<211> 33
<212> DNA
<213> Artificial Sequence

<223> "DNAzym"

<220>
<223> DNAzym

<400> 109
tcacggtcag gctagctaca acgacggcgg gaa                                    33

<210> 110
<211> 33
<212> DNA
<213> Artificial Sequence

<223> "DNAzym"

<220>
<223> DNAzym

<400> 110

gttcgccgag gctagctaca acgacacggt cat                                    33

<210> 111
<211> 33
<212> DNA
<213> Artificial Sequence

<223> "DNAzym"

<220>
<223> DNAzym

<400> 111
cgcggtagag gctagctaca acgattcctc gag                                    33

<210> 112
<211> 33
<212> DNA
<213> Artificial Sequence

<223> "DNAzym"

<220>
<223> DNAzym

<400> 112
tgcggatgag gctagctaca acgacggcgc gtc                                    33

<210> 113
<211> 33
<212> DNA
<213> Artificial Sequence

<223> "DNAzym"

<220>
<223> DNAzym

<400> 113
tcttgcggag gctagctaca acgagatcgg cgc                                    33

<210> 114
<211> 33
<212> DNA
<213> Artificial Sequence

<223> "DNAzym"

<220>
<223> DNAzym

<400> 114
acttgatcag gctagctaca acgactcggc gac                                    33

<210> 115
<211> 33
<212> DNA
<213> Artificial Sequence

<223> "DNAzym"

<220>
<223> DNAzym

<400> 115
tgtacttgag gctagctaca acgacatctc ggc                                    33

<210> 116
<211> 33
<212> DNA
<213> Artificial Sequence

<223> "DNAzym"

<220>
<223> DNAzym

<400> 116
tgttgccgag gctagctaca acgactcgtt ggc                                    33

<210> 117
<211> 33
<212> DNA
<213> Artificial Sequence

<223> "DNAzym"

<220>
<223> DNAzym

<400> 117
ccttggcgag gctagctaca acgagttgcc gat                                    33

<210> 118
<211> 33
<212> DNA
<213> Artificial Sequence

<223> "DNAzym"

<220>
<223> DNAzym

<400> 118
tcacgtccag gctagctaca acgacacctc gcg                                    33



<210> 119
<211> 33
<212> DNA
<213> Artificial Sequence

<223> "DNAzym"

<220>
<223> DNAzym

<400> 119
cctggcagag gctagctaca acgacacgtc cat                                    33



<210> 120
<211> 33
<212> DNA
<213> Artificial Sequence

<223> "DNAzym"

<220>
<223> DNAzym

<400> 120
cgggacgcag gctagctaca acgagtagta gcg                                    33



<210> 121
<211> 33
<212> DNA
<213> Artificial Sequence

<223> "DNAzym"

<220>
<223> DNAzym

<400> 121
gcgcgtacag gctagctaca acgaccttgg gca                                    33

<210> 122
<211> 33
<212> DNA
<213> Artificial Sequence

<223> "DNAzym"

<220>
<223> DNAzym

<400> 122
ctggcgcgag gctagctaca acgaaggtga ggg                                    33

<210> 123
<211> 33
<212> DNA
<213> Artificial Sequence

<223> "DNAzym"

<220>
<223> DNAzym

<400> 123
aaccgagcag gctagctaca acgacgggtg ctc                                    33

<210> 124
<211> 33
<212> DNA
<213> Artificial Sequence

<223> "DNAzym"

<220>
<223> DNAzym

<400> 124
tgctgcgcag gctagctaca acgacaacga acc                                    33

<210> 125

<211> 33
<212> DNA
<213> Artificial Sequence

<223> "DNAzym"

<220>
<223> DNAzym

<400> 125
gtgatgagag gctagctaca acgacgaagg cct                                    33

<210> 126
<211> 33
<212> DNA
<213> Artificial Sequence

<223> "DNAzym"

<220>
<223> DNAzym

<400> 126
ggctggtgag gctagctaca acgagagatc gaa                                    33

<210> 127
<211> 33
<212> DNA
<213> Artificial Sequence

<223> "DNAzym"

<220>
<223> DNAzym

<400> 127
tcgcgcaaag gctagctaca acgacgtcgg tgc                                    33

<210> 128
<211> 33
<212> DNA
<213> Artificial Sequence

<223> "DNAzym"

<220>
<223> DNAzym

<400> 128
cgatgagcag gctagctaca acgactcggc cag                                    33

<210> 129
<211> 33
<212> DNA
<213> Artificial Sequence

<223> "DNAzym"

<220>
<223> DNAzym

<400> 129
ccttgccgag gctagctaca acgagagcat ctc                                    33

<210> 130
<211> 33
<212> DNA
<213> Artificial Sequence

<223> "DNAzym"

<220>
<223> DNAzym

<400> 130
ggtcgaagag gctagctaca acgagcgcaa ctc                                    33

<210> 131
<211> 33
<212> DNA
<213> Artificial Sequence

<223> "DNAzym"

<220>
<223> DNAzym

<400> 131
acgcgcgtag gctagctaca acgatcgacg ttg                                    33

<210> 132
<211> 33
<212> DNA

<213> Artificial Sequence

<223> "DNAzym"

<220>
<223> DNAzym

<400> 132
ctcgatgtag gctagctaca acgatccttg ttg                                33

<210> 133
<211> 33
<212> DNA
<213> Artificial Sequence

<223> "DNAzym"

<220>
<223> DNAzym

<400> 133
tcgactcgag gctagctaca acgagtattc ctt                                33

<210> 134
<211> 33
<212> DNA
<213> Artificial Sequence

<223> "DNAzym"

<220>
<223> DNAzym

<400> 134
cgtgcgggag gctagctaca acgacttcga ctc                                33

<210> 135
<211> 33
<212> DNA
<213> Artificial Sequence

<223> "DNAzym"

<220>
<223> DNAzym

<400> 135

accagcacag gctagctaca acgacggaac tcg                                    33

<210> 136
<211> 33
<212> DNA
<213> Artificial Sequence

<223> "DNAzym"

<220>
<223> DNAzym

<400> 136
tcgtcgccag gctagctaca acgatgccca gca                                    33

<210> 137
<211> 33
<212> DNA
<213> Artificial Sequence

<223> "DNAzym"

<220>
<223> DNAzym

<400> 137
tgtgcggcag gctagctaca acgagaagcc cac                                    33

<210> 138
<211> 33
<212> DNA
<213> Artificial Sequence

<223> "DNAzym"

<220>
<223> DNAzym

<400> 138
accagcagag gctagctaca acgagccctc ggc                                    33

<210> 139
<211> 33
<212> DNA
<213> Artificial Sequence

<223> "DNAzym"

<220>
<223> DNAzym

<400> 139
gctgattcgg gctagctaca acgaatcgca ttc 33

<210> 140
<211> 33
<212> DNA
<213> Artificial Sequence

<223> "DNAzym"

<220>
<223> DNAzym

<400> 140
ccaaagatgg gctagctaca acgatgattc gca 33

<210> 141
<211> 33
<212> DNA
<213> Artificial Sequence

<223> "DNAzym"

<220>
<223> DNAzym

<400> 141
ccgacatagg gctagctaca acgaccaaac caa 33

<210> 142
<211> 33
<212> DNA
<213> Artificial Sequence

<223> "DNAzym"

<220>
<223> DNAzym

<400> 142
cacatactgg gctagctaca acgaaccgac ata 33

<210> 143
<211> 33
<212> DNA
<213> Artificial Sequence

<223> "DNAzym"

<220>
<223> DNAzym

<400> 143
ggcagccagg gctagctaca acgaacatac tgc                                    33



<210> 144
<211> 33
<212> DNA
<213> Artificial Sequence

<223> "DNAzym"

<220>
<223> DNAzym

<400> 144
gacaggcagg gctagctaca acgacagcac ata                                    33



<210> 145
<211> 33
<212> DNA
<213> Artificial Sequence

<223> "DNAzym"

<220>
<223> DNAzym

<400> 145
gccgacaggg gctagctaca acgaagccag cac                                    33



<210> 146
<211> 33
<212> DNA
<213> Artificial Sequence

<223> "DNAzym"

<220>
<223> DNAzym

<400> 146
gaccgcgtgg gctagctaca acgacgacag gca                                    33

<210> 147
<211> 33
<212> DNA
<213> Artificial Sequence

<223> "DNAzym"

<220>
<223> DNAzym

<400> 147
tcatgaccgg gctagctaca acgagtgccg aca                                    33

<210> 148
<211> 33
<212> DNA
<213> Artificial Sequence

<223> "DNAzym"

<220>
<223> DNAzym

<400> 148
atcttggtgg gctagctaca acgatggaga cat                                    33

<210> 149
<211> 33
<212> DNA
<213> Artificial Sequence

<223> "DNAzym"

<220>
<223> DNAzym

<400> 149
ggcgacttgg gctagctaca acgacctggt tga                                    33

<210> 150

<211> 33
<212> DNA
<213> Artificial Sequence

<223> "DNAzym"

<220>
<223> DNAzym

<400> 150
gacgatgggg gctagctaca acgagacttg ccc                                    33


<210> 151
<211> 33
<212> DNA
<213> Artificial Sequence

<223> "DNAzym"

<220>
<223> DNAzym

<400> 151
gcttccaggg gctagctaca acgaccggtt cga                                    33


<210> 152
<211> 33
<212> DNA
<213> Artificial Sequence

<223> "DNAzym"

<220>
<223> DNAzym

<400> 152
gcaacaacgg gctagctaca acgattccag gcc                                    33


<210> 153
<211> 33
<212> DNA
<213> Artificial Sequence

<223> "DNAzym"

<220>
<223> DNAzym

<400> 153
gccttgctgg gctagctaca acgaaacaac gct                                    33

<210> 154
<211> 33
<212> DNA
<213> Artificial Sequence

<223> "DNAzym"

<220>
<223> DNAzym

<400> 154
ccggccttgg gctagctaca acgatgcaac aac                                    33

<210> 155
<211> 33
<212> DNA
<213> Artificial Sequence

<223> "DNAzym"

<220>
<223> DNAzym

<400> 155
gtctgccggg gctagctaca acgacttgct gca                                    33

<210> 156
<211> 33
<212> DNA
<213> Artificial Sequence

<223> "DNAzym"

<220>
<223> DNAzym

<400> 156
tccggtctgg gctagctaca acgacggcct tgc                                    33

<210> 157
<211> 33
<212> DNA

<213> Artificial Sequence

<223> "DNAzym"

<220>
<223> DNAzym

<400> 157
gcccgacagg gctagctaca acgacgtccg gtc                                    33

<210> 158
<211> 33
<212> DNA
<213> Artificial Sequence

<223> "DNAzym"

<220>
<223> DNAzym

<400> 158
tcggtggtgg gctagctaca acgaccgaca gcc                                    33

<210> 159
<211> 33
<212> DNA
<213> Artificial Sequence

<223> "DNAzym"

<220>
<223> DNAzym

<400> 159
ccagcacggg gctagctaca acgacttctt gaa                                    33

<210> 160
<211> 33
<212> DNA
<213> Artificial Sequence

<223> "DNAzym"

<220>
<223> DNAzym

<400> 160

ggagtccagg gctagctaca acgaacggcc ttc 33

<210> 161
<211> 33
<212> DNA
<213> Artificial Sequence

<223> "DNAzym"

<220>
<223> DNAzym

<400> 161
gtgccgacgg gctagctaca acgaagatga acg 33

<210> 162
<211> 33
<212> DNA
<213> Artificial Sequence

<223> "DNAzym"

<220>
<223> DNAzym

<400> 162
ctcggcgtgg gctagctaca acgacgacgc aga 33

<210> 163
<211> 33
<212> DNA
<213> Artificial Sequence

<223> "DNAzym"

<220>
<223> DNAzym

<400> 163
cttgctcggg gctagctaca acgagtgccg acg 33

<210> 164
<211> 33
<212> DNA
<213> Artificial Sequence

<223> "DNAzym"

<220>
<223> DNAzym

<400> 164
ttcttcttgg gctagctaca acgatcggcg tgc 33

<210> 165
<211> 33
<212> DNA
<213> Artificial Sequence

<223> "DNAzym"

<220>
<223> DNAzym

<400> 165
tccaggtcgg gctagctaca acgacgttct tct 33

<210> 166
<211> 33
<212> DNA
<213> Artificial Sequence

<223> "DNAzym"

<220>
<223> DNAzym

<400> 166
cgatgtagcg gctagctaca acgacaggtc cag 33

<210> 167
<211> 33
<212> DNA
<213> Artificial Sequence

<223> "DNAzym"

<220>
<223> DNAzym

<400> 167
atctcgcggg gctagctaca acgaagacgg tct 33

<210> 168
<211> 33
<212> DNA
<213> Artificial Sequence

<223> "DNAzym"

<220>
<223> DNAzym

<400> 168
ccgatctcgg gctagctaca acgaggcaga cgg                                    33



<210> 169
<211> 33
<212> DNA
<213> Artificial Sequence

<223> "DNAzym"

<220>
<223> DNAzym

<400> 169
atggcgaagg gctagctaca acgacgatct cgc                                    33



<210> 170
<211> 33
<212> DNA
<213> Artificial Sequence

<223> "DNAzym"

<220>
<223> DNAzym

<400> 170
cgcggatggg gctagctaca acgagaagcc gat                                    33



<210> 171
<211> 33
<212> DNA
<213> Artificial Sequence

<223> "DNAzym"

<220>
<223> DNAzym

<400> 171
gacttctcgg gctagctaca acgaggatgg cga                                    33

<210> 172
<211> 33
<212> DNA
<213> Artificial Sequence

<223> "DNAzym"

<220>
<223> DNAzym

<400> 172
acggtgtggg gctagctaca acgagttcgg act                                    33

<210> 173
<211> 33
<212> DNA
<213> Artificial Sequence

<223> "DNAzym"

<220>
<223> DNAzym

<400> 173
ggtgctgcgg gctagctaca acgaaccacc acg                                    33

<210> 174
<211> 33
<212> DNA
<213> Artificial Sequence

<223> "DNAzym"

<220>
<223> DNAzym

<400> 174
acggtgctgg gctagctaca acgagcacca cca                                    33

<210> 175

<211> 33
<212> DNA
<213> Artificial Sequence

<223> "DNAzym"

<220>
<223> DNAzym

<400> 175
agtacggtgg gctagctaca acgatgcgca cca                                    33



<210> 176
<211> 33
<212> DNA
<213> Artificial Sequence

<223> "DNAzym"

<220>
<223> DNAzym

<400> 176
ggtgcccggg gctagctaca acgaagtacg gtg                                    33



<210> 177
<211> 33
<212> DNA
<213> Artificial Sequence

<223> "DNAzym"

<220>
<223> DNAzym

<400> 177
ttgacggtgg gctagctaca acgaccggca gta                                    33



<210> 178
<211> 33
<212> DNA
<213> Artificial Sequence

<223> "DNAzym"

<220>
<223> DNAzym

<400> 178
ctcgatcagg gctagctaca acgagggatc acc                                    33

<210> 179
<211> 33
<212> DNA
<213> Artificial Sequence

<223> "DNAzym"

<220>
<223> DNAzym

<400> 179
ttgcccgagg gctagctaca acgaagtcct cga                                    33

<210> 180
<211> 33
<212> DNA
<213> Artificial Sequence

<223> "DNAzym"

<220>
<223> DNAzym

<400> 180
gccttcttgg gctagctaca acgaccgagc agt                                    33

<210> 181
<211> 33
<212> DNA
<213> Artificial Sequence

<223> "DNAzym"

<220>
<223> DNAzym

<400> 181
cgaccccggg gctagctaca acgacttctt gcc                                    33

<210> 182
<211> 33
<212> DNA

<213> Artificial Sequence

<223> "DNAzym"

<220>
<223> DNAzym

<400> 182
gtgccgacgg gctagctaca acgacgaagt cga                                    33

<210> 183
<211> 33
<212> DNA
<213> Artificial Sequence

<223> "DNAzym"

<220>
<223> DNAzym

<400> 183
gggttggtgg gctagctaca acgacgacgc cga                                    33

<210> 184
<211> 33
<212> DNA
<213> Artificial Sequence

<223> "DNAzym"

<220>
<223> DNAzym

<400> 184
gtgctctcgg gctagctaca acgaggagga att                                    33

<210> 185
<211> 33
<212> DNA
<213> Artificial Sequence

<223> "DNAzym"

<220>
<223> DNAzym

<400> 185

atcgcggtgg gctagctaca acgatctcgc gga                                    33

<210> 186
<211> 33
<212> DNA
<213> Artificial Sequence

<223> "DNAzym"

<220>
<223> DNAzym

<400> 186
ccttgatcgg gctagctaca acgaggtgct ctc                                    33

<210> 187
<211> 33
<212> DNA
<213> Artificial Sequence

<223> "DNAzym"

<220>
<223> DNAzym

<400> 187
ggtcatcggg gctagctaca acgagggaag tcg                                    33

<210> 188
<211> 33
<212> DNA
<213> Artificial Sequence

<223> "DNAzym"

<220>
<223> DNAzym

<400> 188
tccagttcgg gctagctaca acgacgatca cgg                                    33

<210> 189
<211> 33
<212> DNA
<213> Artificial Sequence

<223> "DNAzym"

<220>
<223> DNAzym

<400> 189
gccggtctgg gctagctaca acgattgtcc agt                                    33

<210> 190
<211> 33
<212> DNA
<213> Artificial Sequence

<223> "DNAzym"

<220>
<223> DNAzym

<400> 190
agaaggtcgg gctagctaca acgacggtct gct                                    33

<210> 191
<211> 33
<212> DNA
<213> Artificial Sequence

<223> "DNAzym"

<220>
<223> DNAzym

<400> 191
tccagctcgg gctagctaca acgaggtaga ttt                                    33

<210> 192
<211> 33
<212> DNA
<213> Artificial Sequence

<223> "DNAzym"

<220>
<223> DNAzym

<400> 192
cgcgtccagg gctagctaca acgatcgcgg tag                                    33

<210> 193
<211> 33
<212> DNA
<213> Artificial Sequence

<223> "DNAzym"

<220>
<223> DNAzym

<400> 193
tgatcggcgg gctagctaca acgagtccag ctc                                    33

<210> 194
<211> 33
<212> DNA
<213> Artificial Sequence

<223> "DNAzym"

<220>
<223> DNAzym

<400> 194
gatgatcggg gctagctaca acgagcgtcc agc                                    33

<210> 195
<211> 33
<212> DNA
<213> Artificial Sequence

<223> "DNAzym"

<220>
<223> DNAzym

<400> 195
acggtcttgg gctagctaca acgaggatga tcg                                    33

<210> 196
<211> 33
<212> DNA
<213> Artificial Sequence

<223> "DNAzym"

<220>
<223> DNAzym

<400> 196
tcatctcggg gctagctaca acgagacctc gac                                    33

<210> 197
<211> 33
<212> DNA
<213> Artificial Sequence

<223> "DNAzym"

<220>
<223> DNAzym

<400> 197
cagacgttgg gctagctaca acgaaggtgt act                                    33

<210> 198
<211> 33
<212> DNA
<213> Artificial Sequence

<223> "DNAzym"

<220>
<223> DNAzym

<400> 198
ggcggcgtgg gctagctaca acgacagacg ttg                                    33

<210> 199
<211> 33
<212> DNA
<213> Artificial Sequence

<223> "DNAzym"

<220>
<223> DNAzym

<400> 199
ccttggcggg gctagctaca acgagtgcca gac                                    33

<210> 200

<211> 33
<212> DNA
<213> Artificial Sequence

<223> "DNAzym"

<220>
<223> DNAzym

<400> 200
tgaccttggg gctagctaca acgaggcgtg cca                                    33


<210> 201
<211> 33
<212> DNA
<213> Artificial Sequence

<223> "DNAzym"

<220>
<223> DNAzym

<400> 201
tctcgttggg gctagctaca acgagaaggt gac                                    33


<210> 202
<211> 33
<212> DNA
<213> Artificial Sequence

<223> "DNAzym"

<220>
<223> DNAzym

<400> 202
gcgatgttgg gctagctaca acgacgatct cgt                                    33


<210> 203
<211> 33
<212> DNA
<213> Artificial Sequence

<223> "DNAzym"

<220>
<223> DNAzym

<400> 203
ccgccttggg gctagctaca acgagatgtt gcc                                    33

<210> 204
<211> 33
<212> DNA
<213> Artificial Sequence

<223> "DNAzym"

<220>
<223> DNAzym

<400> 204
cgccgaccgg gctagctaca acgacttggc gat                                    33

<210> 205
<211> 33
<212> DNA
<213> Artificial Sequence

<223> "DNAzym"

<220>
<223> DNAzym

<400> 205
ccgtcgacgg gctagctaca acgacgaccg cct                                    33

<210> 206
<211> 33
<212> DNA
<213> Artificial Sequence

<223> "DNAzym"

<220>
<223> DNAzym

<400> 206
acctcgcggg gctagctaca acgacgtcga cgc                                    33

<210> 207
<211> 33
<212> DNA

<213> Artificial Sequence

<223> "DNAzym"

<220>
<223> DNAzym

<400> 207
atcacctcgg gctagctaca acgaggccgt cga                                33

<210> 208
<211> 33
<212> DNA
<213> Artificial Sequence

<223> "DNAzym"

<220>
<223> DNAzym

<400> 208
tggtcctggg gctagctaca acgaagatca cgt                                33

<210> 209
<211> 33
<212> DNA
<213> Artificial Sequence

<223> "DNAzym"

<220>
<223> DNAzym

<400> 209
caggttgagg gctagctaca acgattgtgg tcc                                33

<210> 210
<211> 33
<212> DNA
<213> Artificial Sequence

<223> "DNAzym"

<220>
<223> DNAzym

<400> 210

gtagtagcgg gctagctaca acgagacagg ttg                                    33

<210> 211
<211> 33
<212> DNA
<213> Artificial Sequence

<223> "DNAzym"

<220>
<223> DNAzym

<400> 211
atgtagtagg gctagctaca acgagcgaca ggt                                    33

<210> 212
<211> 33
<212> DNA
<213> Artificial Sequence

<223> "DNAzym"

<220>
<223> DNAzym

<400> 212
gccgggacgg gctagctaca acgaatgtag tag                                    33

<210> 213
<211> 33
<212> DNA
<213> Artificial Sequence

<223> "DNAzym"

<220>
<223> DNAzym

<400> 213
aaggcgaagg gctagctaca acgacgggac gca                                    33

<210> 214
<211> 33
<212> DNA
<213> Artificial Sequence

<223> "DNAzym"

<220>
<223> DNAzym

<400> 214
cgccgaaggg gctagctaca acgagaagcc ggg                                    33

<210> 215
<211> 33
<212> DNA
<213> Artificial Sequence

<223> "DNAzym"

<220>
<223> DNAzym

<400> 215
caggagccgg gctagctaca acgacgaagg cga                                    33

<210> 216
<211> 33
<212> DNA
<213> Artificial Sequence

<223> "DNAzym"

<220>
<223> DNAzym

<400> 216
aggcaggagg gctagctaca acgacgccga agg                                    33

<210> 217
<211> 33
<212> DNA
<213> Artificial Sequence

<223> "DNAzym"

<220>
<223> DNAzym

<400> 217
ttgggcaggg gctagctaca acgaaggagc cgc                                    33

<210> 218
<211> 33
<212> DNA
<213> Artificial Sequence

<223> "DNAzym"

<220>
<223> DNAzym

<400> 218
atccttgggg gctagctaca acgaaggcag gag                                    33

<210> 219
<211> 33
<212> DNA
<213> Artificial Sequence

<223> "DNAzym"

<220>
<223> DNAzym

<400> 219
gtgagggcgg gctagctaca acgagtacat cct                                    33

<210> 220
<211> 33
<212> DNA
<213> Artificial Sequence

<223> "DNAzym"

<220>
<223> DNAzym

<400> 220
aggtgagggg gctagctaca acgagcgtac atc                                    33

<210> 221
<211> 33
<212> DNA
<213> Artificial Sequence

<223> "DNAzym"

<220>
<223> DNAzym

<400> 221
tggctggcgg gctagctaca acgagatagg tga                                    33

<210> 222
<211> 33
<212> DNA
<213> Artificial Sequence

<223> "DNAzym"

<220>
<223> DNAzym

<400> 222
gctggctggg gctagctaca acgagcgata ggt                                    33

<210> 223
<211> 33
<212> DNA
<213> Artificial Sequence

<223> "DNAzym"

<220>
<223> DNAzym

<400> 223
tccagctggg gctagctaca acgatggcgc gat                                    33

<210> 224
<211> 33
<212> DNA
<213> Artificial Sequence

<223> "DNAzym"

<220>
<223> DNAzym

<400> 224
gacgtccagg gctagctaca acgatggctg gcg                                    33

<210> 225

<211> 33
<212> DNA
<213> Artificial Sequence

<223> "DNAzym"

<220>
<223> DNAzym

<400> 225
catcgggtgg gctagctaca acgatcgacg tcc                                    33


<210> 226
<211> 33
<212> DNA
<213> Artificial Sequence

<223> "DNAzym"

<220>
<223> DNAzym

<400> 226
cgaaccgagg gctagctaca acgaatcggg tgc                                    33


<210> 227
<211> 33
<212> DNA
<213> Artificial Sequence

<223> "DNAzym"

<220>
<223> DNAzym

<400> 227
gttgctgcgg gctagctaca acgaatcaac gaa                                    33


<210> 228
<211> 33
<212> DNA
<213> Artificial Sequence

<223> "DNAzym"

<220>
<223> DNAzym

<400> 228
gagttgctgg gctagctaca acgagcatca acg                                    33

<210> 229
<211> 33
<212> DNA
<213> Artificial Sequence

<223> "DNAzym"

<220>
<223> DNAzym

<400> 229
ttggagttgg gctagctaca acgatgcgca tca                                    33

<210> 230
<211> 33
<212> DNA
<213> Artificial Sequence

<223> "DNAzym"

<220>
<223> DNAzym

<400> 230
ggccttctgg gctagctaca acgaacctgg ttg                                    33

<210> 231
<211> 33
<212> DNA
<213> Artificial Sequence

<223> "DNAzym"

<220>
<223> DNAzym

<400> 231
gatcgaaggg gctagctaca acgacttctg cac                                    33

<210> 232
<211> 33
<212> DNA

<213> Artificial Sequence

<223> "DNAzym"

<220>
<223> DNAzym

<400> 232
gtgtcgtggg gctagctaca acgatggtga tga                                33

<210> 233
<211> 33
<212> DNA
<213> Artificial Sequence

<223> "DNAzym"

<220>
<223> DNAzym

<400> 233
ccgaccttgg gctagctaca acgagggtgt cgt                                33

<210> 234
<211> 33
<212> DNA
<213> Artificial Sequence

<223> "DNAzym"

<220>
<223> DNAzym

<400> 234
ccgagcaggg gctagctaca acgacgacct tgc                                33

<210> 235
<211> 33
<212> DNA
<213> Artificial Sequence

<223> "DNAzym"

<220>
<223> DNAzym

<400> 235

caggccgagg gctagctaca acgaaggccg acc                                    33

<210> 236
<211> 33
<212> DNA
<213> Artificial Sequence

<223> "DNAzym"

<220>
<223> DNAzym

<400> 236
aacgacaggg gctagctaca acgacgagca ggc                                    33

<210> 237
<211> 33
<212> DNA
<213> Artificial Sequence

<223> "DNAzym"

<220>
<223> DNAzym

<400> 237
cggtgccggg gctagctaca acgacttgaa cga                                    33

<210> 238
<211> 33
<212> DNA
<213> Artificial Sequence

<223> "DNAzym"

<220>
<223> DNAzym

<400> 238
tcgtcggtgg gctagctaca acgacggcct tga                                    33

<210> 239
<211> 33
<212> DNA
<213> Artificial Sequence

<223> "DNAzym"

<220>
<223> DNAzym

<400> 239
gctttcgcgg gctagctaca acgaaaatcg tcg 33

<210> 240
<211> 33
<212> DNA
<213> Artificial Sequence

<223> "DNAzym"

<220>
<223> DNAzym

<400> 240
gggctttcgg gctagctaca acgagcaaat cgt 33

<210> 241
<211> 33
<212> DNA
<213> Artificial Sequence

<223> "DNAzym"

<220>
<223> DNAzym

<400> 241
accagcgggg gctagctaca acgatttcgc gca 33

<210> 242
<211> 33
<212> DNA
<213> Artificial Sequence

<223> "DNAzym"

<220>
<223> DNAzym

<400> 242
ctccaccagg gctagctaca acgagggctt tcg 33

<210> 243
<211> 33
<212> DNA
<213> Artificial Sequence

<223> "DNAzym"

<220>
<223> DNAzym

<400> 243
ctcggccagg gctagctaca acgatccacc agc                                    33

<210> 244
<211> 33
<212> DNA
<213> Artificial Sequence

<223> "DNAzym"

<220>
<223> DNAzym

<400> 244
gcatctcggg gctagctaca acgacagctc cac                                    33

<210> 245
<211> 33
<212> DNA
<213> Artificial Sequence

<223> "DNAzym"

<220>
<223> DNAzym

<400> 245
gccgatgagg gctagctaca acgaatctcg gcc                                    33

<210> 246
<211> 33
<212> DNA
<213> Artificial Sequence

<223> "DNAzym"

<220>
<223> DNAzym

<400> 246
tagcccttgg gctagctaca acgacgatga gca                                    33

<210> 247
<211> 33
<212> DNA
<213> Artificial Sequence

<223> "DNAzym"

<220>
<223> DNAzym

<400> 247
aactcgtagg gctagctaca acgaccttgc cga                                    33

<210> 248
<211> 33
<212> DNA
<213> Artificial Sequence

<223> "DNAzym"

<220>
<223> DNAzym

<400> 248
gaagatgcgg gctagctaca acgaaactcg tag                                    33

<210> 249
<211> 33
<212> DNA
<213> Artificial Sequence

<223> "DNAzym"

<220>
<223> DNAzym

<400> 249
tcgaagatgg gctagctaca acgagcaact cgt                                    33

<210> 250

<211> 33
<212> DNA
<213> Artificial Sequence

<223> "DNAzym"

<220>
<223> DNAzym

<400> 250
tcgacgttgg gctagctaca acgaggtcga aga                                    33


<210> 251
<211> 33
<212> DNA
<213> Artificial Sequence

<223> "DNAzym"

<220>
<223> DNAzym

<400> 251
ggacacgcgg gctagctaca acgagtattc gac                                    33


<210> 252
<211> 33
<212> DNA
<213> Artificial Sequence

<223> "DNAzym"

<220>
<223> DNAzym

<400> 252
gtggacacgg gctagctaca acgagcgtat tcg                                    33


<210> 253
<211> 33
<212> DNA
<213> Artificial Sequence

<223> "DNAzym"

<220>
<223> DNAzym

<400> 253
ccttgttggg gctagctaca acgacccgtg gac                                    33

<210> 254
<211> 33
<212> DNA
<213> Artificial Sequence

<223> "DNAzym"

<220>
<223> DNAzym

<400> 254
ggagacgtgg gctagctaca acgagggatc ttc                                    33

<210> 255
<211> 33
<212> DNA
<213> Artificial Sequence

<223> "DNAzym"

<220>
<223> DNAzym

<400> 255
ggagaccagg gctagctaca acgaagtgag gag                                    33

<210> 256
<211> 33
<212> DNA
<213> Artificial Sequence

<223> "DNAzym"

<220>
<223> DNAzym

<400> 256
cggaactcgg gctagctaca acgacaccac ttc                                    33

<210> 257
<211> 33
<212> DNA

<213> Artificial Sequence

<223> "DNAzym"

<220>
<223> DNAzym

<400> 257
cagcaccagg gctagctaca acgaacatcg gaa                                    33

<210> 258
<211> 33
<212> DNA
<213> Artificial Sequence

<223> "DNAzym"

<220>
<223> DNAzym

<400> 258
attgcccagg gctagctaca acgaaccagc aca                                    33

<210> 259
<211> 33
<212> DNA
<213> Artificial Sequence

<223> "DNAzym"

<220>
<223> DNAzym

<400> 259
tcgccattgg gctagctaca acgaccagca cca                                    33

<210> 260
<211> 33
<212> DNA
<213> Artificial Sequence

<223> "DNAzym"

<220>
<223> DNAzym

<400> 260

agctcgtcgg gctagctaca acgacattgc cca                                    33

<210> 261
<211> 33
<212> DNA
<213> Artificial Sequence

<223> "DNAzym"

<220>
<223> DNAzym

<400> 261
gacgaacagg gctagctaca acgatcgtcg cca                                    33

<210> 262
<211> 33
<212> DNA
<213> Artificial Sequence

<223> "DNAzym"

<220>
<223> DNAzym

<400> 262
ttcttgccgg gctagctaca acgatcgggg tct                                    33

<210> 263
<211> 33
<212> DNA
<213> Artificial Sequence

<223> "DNAzym"

<220>
<223> DNAzym

<400> 263
agcttcttgg gctagctaca acgacgctcg ggg                                    33

<210> 264
<211> 33
<212> DNA
<213> Artificial Sequence

<223> "DNAzym"

<220>
<223> DNAzym

<400> 264
gtcgaccagg gctagctaca acgattcttg ccg                                    33

<210> 265
<211> 33
<212> DNA
<213> Artificial Sequence

<223> "DNAzym"

<220>
<223> DNAzym

<400> 265
ggcatgaagg gctagctaca acgaccacca ggt                                    33

<210> 266
<211> 33
<212> DNA
<213> Artificial Sequence

<223> "DNAzym"

<220>
<223> DNAzym

<400> 266
ggtgtgcggg gctagctaca acgaatgaag ccc                                    33

<210> 267
<211> 33
<212> DNA
<213> Artificial Sequence

<223> "DNAzym"

<220>
<223> DNAzym

<400> 267
ggtggtgtgg gctagctaca acgaggcatg aag                                    33

<210> 268
<211> 33
<212> DNA
<213> Artificial Sequence

<223> "DNAzym"

<220>
<223> DNAzym

<400> 268
cggcctgggg gctagctaca acgaagtggt ggt                                    33

<210> 269
<211> 33
<212> DNA
<213> Artificial Sequence

<223> "DNAzym"

<220>
<223> DNAzym

<400> 269
tgccctcggg gctagctaca acgactgggc agt                                    33

<210> 270
<211> 33
<212> DNA
<213> Artificial Sequence

<223> "DNAzym"

<220>
<223> DNAzym

<400> 270
cagcagatgg gctagctaca acgacctcgg cct                                    33

<210> 271
<211> 33
<212> DNA
<213> Artificial Sequence

<223> "DNAzym"

<220>
<223> DNAzym

<400> 271
cgctaccagg gctagctaca acgaagatgc cct                                    33

<210> 272
<211> 33
<212> DNA
<213> Artificial Sequence

<223> "DNAzym"

<220>
<223> DNAzym

<400> 272
tagcccaaag gctagctaca acgacaaaga tgc                                    33

<210> 273
<211> 33
<212> DNA
<213> Artificial Sequence

<223> "DNAzym"

<220>
<223> DNAzym

<400> 273
ctgcaccgag gctagctaca acgaatagcc caa                                    33

<210> 274
<211> 33
<212> DNA
<213> Artificial Sequence

<223> "DNAzym"

<220>
<223> DNAzym

<400> 274
catactgcag gctagctaca acgacgacat agc                                    33

<210> 275

<211> 33
<212> DNA
<213> Artificial Sequence

<223> "DNAzym"

<220>
<223> DNAzym

<400> 275
cagcacatag gctagctaca acgatgcacc gac                                    33


<210> 276
<211> 33
<212> DNA
<213> Artificial Sequence

<223> "DNAzym"

<220>
<223> DNAzym

<400> 276
cagccagcag gctagctaca acgaatactg cac                                    33


<210> 277
<211> 33
<212> DNA
<213> Artificial Sequence

<223> "DNAzym"

<220>
<223> DNAzym

<400> 277
gcgtgccgag gctagctaca acgaaggcag cca                                    33


<210> 278
<211> 33
<212> DNA
<213> Artificial Sequence

<223> "DNAzym"

<220>
<223> DNAzym

<400> 278
acttcatgag gctagctaca acgacgcgtg ccg                                    33

<210> 279
<211> 33
<212> DNA
<213> Artificial Sequence

<223> "DNAzym"

<220>
<223> DNAzym

<400> 279
caccaatgag gctagctaca acgattcatg acc                                    33

<210> 280
<211> 33
<212> DNA
<213> Artificial Sequence

<223> "DNAzym"

<220>
<223> DNAzym

<400> 280
acatccacag gctagctaca acgacaatga ctt                                    33

<210> 281
<211> 33
<212> DNA
<213> Artificial Sequence

<223> "DNAzym"

<220>
<223> DNAzym

<400> 281
agacatccag gctagctaca acgaaccaat gac                                    33

<210> 282
<211> 33
<212> DNA

<213> Artificial Sequence

<223> "DNAzym"

<220>
<223> DNAzym

<400> 282
tgctggagag gctagctaca acgaatccac acc                                33

<210> 283
<211> 33
<212> DNA
<213> Artificial Sequence

<223> "DNAzym"

<220>
<223> DNAzym

<400> 283
gatgggcgag gctagctaca acgattgccc tgg                                33

<210> 284
<211> 33
<212> DNA
<213> Artificial Sequence

<223> "DNAzym"

<220>
<223> DNAzym

<400> 284
ccggttcgag gctagctaca acgagatggg cga                                33

<210> 285
<211> 33
<212> DNA
<213> Artificial Sequence

<223> "DNAzym"

<220>
<223> DNAzym

<400> 285

ctgcaacaag gctagctaca acgagcttcc agg                                    33

<210> 286
<211> 33
<212> DNA
<213> Artificial Sequence

<223> "DNAzym"

<220>
<223> DNAzym

<400> 286
ttgctgcaag gctagctaca acgaaacgct tcc                                    33

<210> 287
<211> 33
<212> DNA
<213> Artificial Sequence

<223> "DNAzym"

<220>
<223> DNAzym

<400> 287
ggtgcccgag gctagctaca acgaagccgt ccg                                    33

<210> 288
<211> 33
<212> DNA
<213> Artificial Sequence

<223> "DNAzym"

<220>
<223> DNAzym

<400> 288
agtccagcag gctagctaca acgaggcctt ctt                                    33

<210> 289
<211> 33
<212> DNA
<213> Artificial Sequence

<223> "DNAzym"

<220>
<223> DNAzym

<400> 289
tgaacgatag gctagctaca acgagtcgga gtc                                    33

<210> 290
<211> 33
<212> DNA
<213> Artificial Sequence

<223> "DNAzym"

<220>
<223> DNAzym

<400> 290
gcagatgaag gctagctaca acgagatacg tcg                                    33

<210> 291
<211> 33
<212> DNA
<213> Artificial Sequence

<223> "DNAzym"

<220>
<223> DNAzym

<400> 291
gcgtgccgag gctagctaca acgagcagat gaa                                    33

<210> 292
<211> 33
<212> DNA
<213> Artificial Sequence

<223> "DNAzym"

<220>
<223> DNAzym

<400> 292
cgcggcagag gctagctaca acgaggtctc gat                                    33

<210> 293
<211> 33
<212> DNA
<213> Artificial Sequence

<223> "DNAzym"

<220>
<223> DNAzym

<400> 293
gcgttcggag gctagctaca acgattctcg cgg                                    33

<210> 294
<211> 33
<212> DNA
<213> Artificial Sequence

<223> "DNAzym"

<220>
<223> DNAzym

<400> 294
gcaccaccag gctagctaca acgaggtgtg gcg                                    33

<210> 295
<211> 33
<212> DNA
<213> Artificial Sequence

<223> "DNAzym"

<220>
<223> DNAzym

<400> 295
tgcgcaccag gctagctaca acgacacggt gtg                                    33

<210> 296
<211> 33
<212> DNA
<213> Artificial Sequence

<223> "DNAzym"

<220>
<223> DNAzym

<400> 296
tgctgcgcag gctagctaca acgacaccac ggt                                    33

<210> 297
<211> 33
<212> DNA
<213> Artificial Sequence

<223> "DNAzym"

<220>
<223> DNAzym

<400> 297
ccggcagtag gctagctaca acgaggtgct gcg                                    33

<210> 298
<211> 33
<212> DNA
<213> Artificial Sequence

<223> "DNAzym"

<220>
<223> DNAzym

<400> 298
cgttgttgag gctagctaca acgaggtgcc cgg                                    33

<210> 299
<211> 33
<212> DNA
<213> Artificial Sequence

<223> "DNAzym"

<220>
<223> DNAzym

<400> 299
ggatcaccag gctagctaca acgagttgtt gac                                    33

<210> 300

<211> 33
<212> DNA
<213> Artificial Sequence

<223> "DNAzym"

<220>
<223> DNAzym

<400> 300
gcgggatcag gctagctaca acgacacgtt gtt                                    33

<210> 301
<211> 33
<212> DNA
<213> Artificial Sequence

<223> "DNAzym"

<220>
<223> DNAzym

<400> 301
cgaagtcgag gctagctaca acgacccggc ctt                                    33

<210> 302
<211> 33
<212> DNA
<213> Artificial Sequence

<223> "DNYzym"

<220>
<223> DNYzym

<400> 302
tggtgccgag gctagctaca acgagccgaa gtc                                    33

<210> 303
<211> 33
<212> DNA
<213> Artificial Sequence

<223> "DNAzym"

<220>
<223> DNAzym

<400> 303
cgccgatcag gctagctaca acgaggtcat cgg                                    33

<210> 304
<211> 33
<212> DNA
<213> Artificial Sequence

<223> "DNAzym"

<220>
<223> DNAzym

<400> 304
cgacctcgag gctagctaca acgaggtctt gcg                                    33

<210> 305
<211> 33
<212> DNA
<213> Artificial Sequence

<223> "DNAzym"

<220>
<223> DNAzym

<400> 305
tctcggcgag gctagctaca acgactcgac ggt                                    33

<210> 306
<211> 33
<212> DNA
<213> Artificial Sequence

<223> "DNAzym"

<220>
<223> DNAzym

<400> 306
gcaggtgtag gctagctaca acgattgatc atc                                    33

<210> 307
<211> 33
<212> DNA

<213> Artificial Sequence

<223> "DNAzym"

<220>
<223> DNAzym

<400> 307
cgtgccagag gctagctaca acgagttgca ggt                                    33

<210> 308
<211> 33
<212> DNA
<213> Artificial Sequence

<223> "DNAzym"

<220>
<223> DNAzym

<400> 308
cgaaggtgag gctagctaca acgacttggc ggc                                    33

<210> 309
<211> 33
<212> DNA
<213> Artificial Sequence

<223> "DNAzym"

<220>
<223> DNAzym

<400> 309
cgacgccgag gctagctaca acgacgcctt ggc                                    33

<210> 310
<211> 33
<212> DNA
<213> Artificial Sequence

<223> "DNAzym"

<220>
<223> DNAzym

<400> 310

ggccgtcgag gctagctaca acgagccgac cgc 33

<210> 311
<211> 33
<212> DNA
<213> Artificial Sequence

<223> "DNAzym"

<220>
<223> DNAzym

<400> 311
cgtccatcag gctagctaca acgactcgcg gcc 33

<210> 312
<211> 33
<212> DNA
<213> Artificial Sequence

<223> "DNAzym"

<220>
<223> DNAzym

<400> 312
ggcagatcag gctagctaca acgagtccat cac 33

<210> 313
<211> 33
<212> DNA
<213> Artificial Sequence

<223> "DNAzym"

<220>
<223> DNAzym

<400> 313
gtagcgcgag gctagctaca acgaaggttg agc 33

<210> 314
<211> 33
<212> DNA
<213> Artificial Sequence

<223> "DNAzym"

<220>
<223> DNAzym

<400> 314
aagccgggag gctagctaca acgagcatgt agt                                    33

<210> 315
<211> 33
<212> DNA
<213> Artificial Sequence

<223> "DNAzym"

<220>
<223> DNAzym

<400> 315
gggcgcgtag gctagctaca acgaatcctt ggg                                    33

<210> 316
<211> 33
<212> DNA
<213> Artificial Sequence

<223> "DNAzym"

<220>
<223> DNAzym

<400> 316
ggtgctcgag gctagctaca acgagtccag ctg                                    33

<210> 317
<211> 33
<212> DNA
<213> Artificial Sequence

<223> "DNAzym"

<220>
<223> DNAzym

<400> 317
atcaacgaag gctagctaca acgacgagca tcg                                    33

<210> 318
<211> 33
<212> DNA
<213> Artificial Sequence

<223> "DNAzym"

<220>
<223> DNAzym

<400> 318
gcgcatcaag gctagctaca acgagaaccg agc                                    33



<210> 319
<211> 33
<212> DNA
<213> Artificial Sequence

<223> "DNAzym"

<220>
<223> DNAzym

<400> 319
ccttctgcag gctagctaca acgactggtt gga                                    33



<210> 320
<211> 33
<212> DNA
<213> Artificial Sequence

<223> "DNAzym"

<220>
<223> DNAzym

<400> 320
gcaggccgag gctagctaca acgacttgcg ggt                                    33



<210> 321
<211> 33
<212> DNA
<213> Artificial Sequence

<223> "DNAzym"

<220>
<223> DNAzym

<400> 321
cttgaacgag gctagctaca acgaaggccg agc                                    33

<210> 322
<211> 33
<212> DNA
<213> Artificial Sequence

<223> "DNAzym"

<220>
<223> DNAzym

<400> 322
ggccttgaag gctagctaca acgagacagg ccg                                    33

<210> 323
<211> 33
<212> DNA
<213> Artificial Sequence

<223> "DNAzym"

<220>
<223> DNAzym

<400> 323
ccagctccag gctagctaca acgacagcgg gct                                    33

<210> 324
<211> 33
<212> DNA
<213> Artificial Sequence

<223> "DNAzym"

<220>
<223> DNAzym

<400> 324
gatgcgcaag gctagctaca acgatcgtag ccc                                    33

<210> 325

<211> 33
<212> DNA
<213> Artificial Sequence

<223> "DNAzym"

<220>
<223> DNAzym

<400> 325
cgtattcgag gctagctaca acgagttgcg gtc                                    33


<210> 326
<211> 33
<212> DNA
<213> Artificial Sequence

<223> "DNAzym"

<220>
<223> DNAzym

<400> 326
ccgtggacag gctagctaca acgagcgcgt att                                    33


<210> 327
<211> 33
<212> DNA
<213> Artificial Sequence

<223> "DNAzym"

<220>
<223> DNAzym

<400> 327
ccccgtggag gctagctaca acgaacgcgc gta                                    33


<210> 328
<211> 33
<212> DNA
<213> Artificial Sequence

<223> "DNAzym"

<220>
<223> DNAzym

<400> 328
gatcttcgag gctagctaca acgatcgatg tat                                    33

<210> 329
<211> 33
<212> DNA
<213> Artificial Sequence

<223> "DNAzym"

<220>
<223> DNAzym

<400> 329
gtgaggagag gctagctaca acgagtgcgg gat                                    33

<210> 330
<211> 33
<212> DNA
<213> Artificial Sequence

<223> "DNAzym"

<220>
<223> DNAzym

<400> 330
ggtcggagag gctagctaca acgacagcag tga                                    33

<210> 331
<211> 33
<212> DNA
<213> Artificial Sequence

<223> "DNAzym"

<220>
<223> DNAzym

<400> 331
tcgccaccag gctagctaca acgattcgtc gag                                    33

<210> 332
<211> 33
<212> DNA

<213> Artificial Sequence

<223> "DNAzym"

<220>
<223> DNAzym

<400> 332
aactcgccag gctagctaca acgacacttc gtc                                    33

<210> 333
<211> 33
<212> DNA
<213> Artificial Sequence

<223> "DNAzym"

<220>
<223> DNAzym

<400> 333
acatcggaag gctagctaca acgatcgcca cca                                    33

<210> 334
<211> 33
<212> DNA
<213> Artificial Sequence

<223> "DNAzym"

<220>
<223> DNAzym

<400> 334
gcaccagcag gctagctaca acgaatcgga act                                    33

<210> 335
<211> 33
<212> DNA
<213> Artificial Sequence

<223> "DNAzym"

<220>
<223> DNAzym

<400> 335

tgcccagcag gctagctaca acgacagcac atc                                    33

<210> 336
<211> 33
<212> DNA
<213> Artificial Sequence

<223> "DNAzym"

<220>
<223> DNAzym

<400> 336
gtcgacgaag gctagctaca acgaagctcg tcg                                    33

<210> 337
<211> 33
<212> DNA
<213> Artificial Sequence

<223> "DNAzym"

<220>
<223> DNAzym

<400> 337
ccaggtcgag gctagctaca acgagaacag ctc                                    33

<210> 338
<211> 33
<212> DNA
<213> Artificial Sequence

<223> "DNAzym"

<220>
<223> DNAzym

<400> 338
tcttgttcag gctagctaca acgacaggtc gac                                    33

<210> 339
<211> 33
<212> DNA
<213> Artificial Sequence

<223> "DNAzym"

<220>
<223> DNAzym

<400> 339
ccaggtcgag gctagctaca acgacagctt ctt                                    33

<210> 340
<211> 33
<212> DNA
<213> Artificial Sequence

<223> "DNAzym"

<220>
<223> DNAzym

<400> 340
tgaagcccag gctagctaca acgacaggtc gac                                    33

<210> 341
<211> 33
<212> DNA
<213> Artificial Sequence

<223> "DNAzym"

<220>
<223> DNAzym

<400> 341
ggcccgctag gctagctaca acgacagcag atg                                    33

<210> 342
<211> 1311
<212> DNA
<213> Pseudomonas aeruginosa

<400> 342
atgcgaatca gcatctttgg tttgggctat gtcggtgcag tatgtgctgg ctgcctgtcg      60

gcacgcggtc atgaagtcat tggtgtggat gtctccagca ccaagatcga cctgatcaac     120

cagggcaart cgcccatcgt cgaaccgggc ctggaagygt tgttgcagca aggccggcag     180

accggacggc tktcgggcac caccgayttc aagaaggcyg tgctggactc cgacgtatcg 240

ttcatctgcg tcggcacgcc gagcaagaag aacggcgacc tggacctggg ctacatcgag 300

acygtctgcc gcgagatcgg cttcgccatc cgcgagaagt ccgaacgcca caccgtrgtg 360

gtgcgcagca cygtrctgcc gggcaccgtc aacaacgtgg tgatcccgct ratcgaggac 420

tgctcgggca agaaggccgg ggtcgacttc ggcgtcggca ccaaccccga rttcctccgc 480

gagagcaccg csatcaagga ctacgacttc ccgccgatga ccgtgatcgg cgaactggac 540

aagcagaccg gcgacctkct cgaggaaatc taccgcgagc tggacgcgcc gatcrtccgc 600

aagaccgtcg aggtcgccga gatgatcaar tacacctgca acgtctggca cgccgccaag 660

gtcaccttcg ccaacgagat cggcaacatc gccaaggcgg tcggcgtcga cggccgcgag 720

gtgatggacg tgatctgcca ggaccacaag ctyaacctgt cgcgctacta catgcgtccc 780

ggcttcgcct tcggcggctc ctgcctgccc aaggatgtac gcgccctcac ctatcgcgcc 840

agccagytgg acgtcgagca cccgatgctc ggttcgytga tgcgcagcaa ctccaaccag 900

gtgcagaagg ccttcgayct catcaccagc cacgacaccc gcaaggtcgg yctgctcggc 960

ctgtcgttca aggccggcac cgacgayytg cgcgaaagyc cgctggtgga gctggccgag 1020

atgctcatcg gcaagggcta cgagytgcgc atcttcgacc gcaaygtcga atacgcrcgt 1080

gtccacgggg ccaacaagga atacatcgag tcgaagatcc cgcrcgtctc ctcrytgctg 1140

gtctccgacc tcgacgaagt ggtggcgagt tccgaygtgc tggtgctggg caatggygac 1200

gagctrttcg tcgacctggt garcaagacc ccgagcggca agaagctggt cgacctggtg 1260

ggcttcatgc cgcacaccac cactgcccag gccgagggca tctgctggta g 1311